# FASCICULE DE BREVET EUROPEEN

(11) **EP 0 527 117 B1**
(45) Date de publication et mention de la délivrance du brevet: **29.10.1997**
(21) Numéro de dépôt: 92870101.0
(22) Date de dépôt: 09.07.1992
(51) Int. Cl.: C07D 251/18, C07D 401/04, C07D 417/04, C07D 498/08, A61K 31/53

(54) **Cyclopropylamino-1,3,5-triazines substituées**
Substituierte Cyclopropylamino-1,3,5-Triazine
Substituted cyclopropylamino-1,3,5-triazines

(30) Priorité: 25.07.1991 GB 9116039
(43) Date de publication de la demande: 10.02.1993
(73) Titulaire: U C B, S.A., 1050 Bruxelles (BE)
(72) Inventeur: van Keulen, Berend Jan, B-1480 Tubize (BE); Golstein, Solo, Montreal H3S 1W4 (CA); Cossement, Eric, B-1050 Bruxelles (BE); Gobert, Jean, B-1040 Bruxelles (BE); Wülfert, Ernst, B-1180 Bruxelles (BE)
(74) Mandataire: Debled, Thierry

(56) Documents cités:
- EP-A- 0 356 413

## Description

La présente invention se rapporte à de nouvelles cyclopropylamino-1,3,5-triazines substituées, à leurs sels d'addition d'acides non toxiques pharmaceutiquement acceptables ainsi qu'à des procédés pour les préparer. Elle concerne également les compositions thérapeutiques renfermant lesdits composés.

Selon la demande de brevet japonais 25786/78, on connaît déjà des 2-trifluorométhyl -1,3,5-triazines portant en position 4 entre autres un radical alkyle, alkylamino substitué ou non, dialkylamino, cycloalkylamino, morpholino ou 4-alkyl-1-pipérazinyle, et en position 6 les mêmes radicaux à l'exception du radical alkyle. Selon cette demande de brevet, ces composés ont des propriétés tranquillisantes et anticonvulsantes.

Par ailleurs, dans GB-A-1.053.113, on décrit des 1,2-dihydro-1-hydroxy-1,3,5-triazines portant en position 2 un radical imino éventuellement substitué par un radical alkyle, alkényle, cycloalkyle, phényle ou naphtyle (éventuellement substitué par un radical alkyle), en position 4, un radical dialkylamino, dialkénylamino, N-alkyl-alkénylamino, aziridinyle, azétidinyle, pyrrolidinyle, pipéridino, hexahydroazépinyle, heptaméthylèneimino, octaméthylèneimino ou morpholino, chacun desdits hétérocycles pouvant être substitué par un à trois radicaux alkyles, et en position 6, un atome d'hydrogène ou un radical alkyle, alkényle, alkoxyalkyle, cycloalkyle, phényle ou naphtyle, éventuellement substitué par un radical alkyle, aralkyle, alkylaralkyle, alkoxyaralkyle ou halogénoaralkyle. Selon ce brevet, ces composés sont des agents antihypertensifs utiles pour le traitement de l'hypertension et des états de choc ; ce sont aussi des inhibiteurs des sécrétions et des dépresseurs du système nerveux central. Ces composés sont préparés par oxydation des 1,3,5-triazines correspondantes portant les mêmes substituants dans les positions 2, 4 et 6. Toutefois, il n'est pas indiqué dans ce brevet que les 1,3,5-triazines intermédiaires possèdent une quelconque activité pharmacologique. De plus, ce brevet ne décrit aucune 1,3,5-triazine substituée par un radical cyclopropylamino.

Enfin, dans la demande EP-A-356.413 au nom de la demanderesse, on décrit des 2-amino-4-morpholino-6-propyl-1,3,5-triazines dans lesquelles le groupe amino en position 2 est substitué par divers radicaux comme par exemple un radical hydroxyle ou hydroxyalkyle. Ces composés sont utiles dans le traitement des troubles cognitifs et comportementaux associés à l'âge et aux syndromes démentiels, par exemple ceux associés à la maladie d'Alzheimer. Toutefois, dans cette demande de brevet on ne décrit pas des 1,3,5-triazines substituées par un radical cyclopropylamino.

Poursuivant ses travaux de recherche dans ce domaine, la demanderesse a maintenant trouvé de nouvelles 1,3,5-triazines substituées par un radical cyclopropylamino, qui possèdent des propriétés pharmaceutiques de valeur et en particulier la propriété de favoriser l'apprentissage et d'atténuer l'effet amnésiant résultant d'un hypofonctionnement cholinergique induit par un antagoniste cholinergique tel que, par exemple, la scopolamine. Le système cholinergique est largement impliqué dans les phénomènes de mémorisation et d'apprentissage. Ainsi, par exemple, l'administration à des sujets jeunes d'un agent anticholinergique tel que la scopolamine fait apparaître des déficiences de la mémoire semblables à celles observées chez des sujets âgés. Inversement, des agents cholinergiques, comme la physostigmine, sont capables de combattre l'amnésie résultant de l'administration d'agents anticholinergiques (S.D. GLICK et al., Behavioral Biology,7,(1972),245-254; U. SCHINDLER et al., Drug Develop.Res.,4,(1984),567-576). C'est pourquoi, les composés de l'invention peuvent être utilisés pour le traitement des troubles cognitifs et comportementaux associés à l'âge et aux syndromes démentiels. En particulier, ils trouvent une application dans le traitement des troubles associés à la maladie d'Alzheimer, aux démences séniles de type Alzheimer et à toute pathologie cognitive évolutive (C.G. GOTTFRIES, Psychopharmacology,86, (1985),245-252; C.G. GOTTFRIES, Neurobiology of Ageing,4,(1983),261-271).
Les composés de la présente invention possèdent également une activité sérotonergique centrale, mise en évidence par le pouvoir qu'ils ont d'induire chez le rat une stéréotypie particulière désignée habituellement sous le nom "Wet Dog Shake" (A.R. GREEN et D.J. HEAL, dans "Neuropharmacology of Serotonin", Ed. A.R. GREEN, Oxford Univ. Press, 1985, chapitre 12, pages 326 à 365). Il est connu que la sérotonine joue un rôle important dans la régulation de la fonction neuroendocrine qui peut être perturbée dans des pathologies telles que la dépression, l'anxiété et les troubles de l'humeur. Une diminution de l'activité sérotonergique est reliée à de nombreux changements de l'humeur et des fonctions somatiques survenant chez des patients déprimés (H.Y. MELTZER et M.T. LOWY dans "Psychopharmacology: The Third Generation of Progress", Ed. H.Y. MELTZER, Raven Press, New-York, 1987, chapitre 52, pages 513 à 520). Les composés de l'invention peuvent donc être utilisés pour le traitement de ces diverses pathologies liées à un ralentissement de l'activité sérotonergique.

En outre, au niveau périphérique, les composés de l'invention présentent aussi une activité bronchospasmolytique et inhibitrice de la libération des médiateurs des mastocytes lors d'une agression anaphylactique. De plus, les composés de l'invention potentialisent l'effet de relâchement musculaire d'un agoniste β-adrénergique (par exemple l'isoprénaline) sur un muscle lisse contracté par l'histamine et possèdent aussi une activité antiinflammatoire et antioedème. C'est pourquoi, les composés de l'invention trouvent également une application dans le traitement des phénomènes inflammatoires et de l'asthme, en particulier comme une alternative à un traitement par la théophylline ou même par des agents bronchodilatateurs tels que les agents β-sympatomimétiques , dont on sait qu'ils provoquent, lors de l'administration prolongée, une désensibilisation des récepteurs β-adrénergiques des bronches, au point de rendre le bronchospasme des asthmatiques chroniques insensible et irréversible à l'action de ces agents.

Plus particulièrement, la présente invention se rapporte à de nouvelles cyclopropylamino-1,3,5-triazines substituées répondant à la formule générale 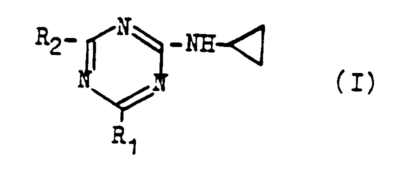 dans laquelle
- R₁: représente un radical alkyle ou un radical cycloalkyle substitué ou non par au moins un radical alkyle, de préférence par un ou deux radicaux alkyle, les radicaux alkyle ayant de 1 à 3 atomes de carbone et le radical cycloalkyle ayant de 3 à 5 atomes de carbone, et
- R₂: représente le radical bis(2-hydroxyéthyl)amino, 3-hydroxy-1-azetidinyle, 3-méthoxy-1-azétidinyle, 3-oxo-1-azétidinyle, morpholino, 4-hydroxypiperidino, thiomorpholino, thiomorpholino S-oxyde, thiomorpholino S,S-dioxyde, 3-thiazolidinyle, 3-thiazolidinyle S-oxyde, 3-thiazolidinyle S,S-dioxyde ou 8-oxa-3-azabicyclo[3,2,1]oct-3-yle,
ainsi que leurs sels d'addition d'acides non toxiques pharmaceutiquement acceptables.

Les composés préférés de la présente invention sont les cyclopropylamino-1,3,5-triazines répondant à la formule générale I, dans laquelle R₂ représente le radical morpholino, thiomorpholino ou thiomorpholino S,S-dioxyde, ainsi que leurs sels d'addition d'acides non toxiques pharmaceutiquement acceptables.

Les composés particulièrement préférés conformes à l'invention sont :
- la 2-cyclopropylamino-4-morpholino-6-n-propyl-1,3,5-triazine,
- le chlorhydrate de 2-cyclopropyl-4-cyclopropylamino-6-morpholino-1,3,5-triazine,
- la 2-cyclobutyl-4-cyclopropylamino-6-morpholino-1,3,5-triazine,
- la 2-cyclopropyl-4-cyclopropylamino-6-thiomorpholino-1,3,5-triazine et
- la 2-cyclopropyl-4-cyclopropylamino-6-thiomorpholino-1,3,5-triazine S,S-dioxyde.

La présente invention concerne également les sels d'addition d'acides non toxiques pharmaceutiquement acceptables des cyclopropylamino-1,3,5-triazines substituées de formule I. Comme exemples d'acides pharmaceutiquement acceptables, on peut citer les acides minéraux comme les acides chlorhydrique, bromhydrique, sulfurique, nitrique, phosphorique, etc., et les acides organiques comme les acides acétique, citrique, tartrique, benzoïque, salicylique, maléique, etc.

Lorsque la molécule comporte un ou plusieurs centres d'asymétrie, les composés de formule I peuvent se présenter soit sous la forme d'un mélange racémique, soit sous la forme de l'un ou l'autre enantiomère. Ces diverses formes entrent également dans le cadre de la présente invention.

Les cyclopropylamino-1,3,5-triazines substituées conformes à la présente invention peuvent être préparées par l'un ou l'autre des procédés suivants :
(a) On fait réagir une chloro-cyclopropylamino-1,3,5-triazine de formule II avec une amine de formule R₂H (III), selon l'équation 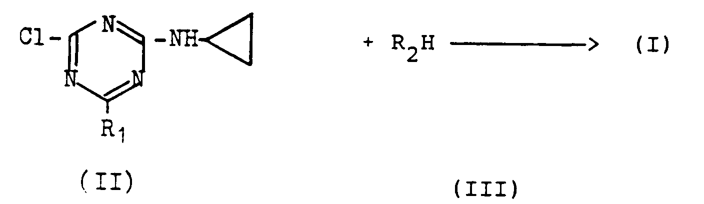 dans ces formules R₁ et R₂ ont les significations données ci-dessus.
(b) On fait réagir une chloro-1,3,5-triazine de formule IV avec la cyclopropylamine de formule V, selon l'équation 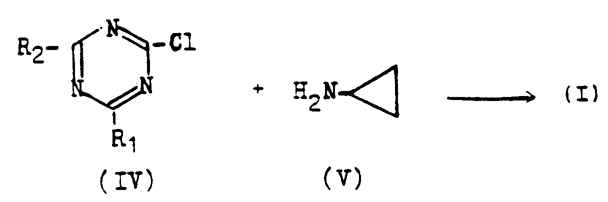 dans ces formules R₁ et R₂ ont les significations données ci-dessus.
(c) On fait réagir par chauffage à reflux dans un alcool aliphatique, pendant plusieurs heures, un N-cyclopropylbiguanide de formule VI avec un ester d'alkyle de formule VII, en présence d'un alcoolate de métal alcalin, selon l'équation 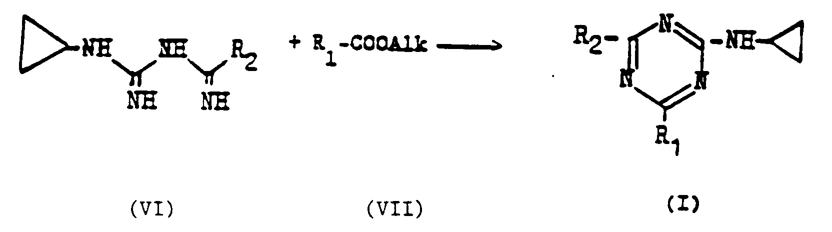 dans ces formules R₁ et R₂ ont la signification donnée ci-dessus et alk représente un radical alkyle ayant de 1 à 4 atomes de carbone, de préference le radical éthyle.
(d) On oxyde une cyclopropylamino-1,3,5-triazine de formule I dans laquelle R₁ a la signification donnée ci-dessus et R₂ représente le radical thiomorpholino ou 3-thiazolidinyle pour préparer les cyclopropylamino-1,3,5-triazines de formule I, dans laquelle R₂ représente le radical thiomorpholino S-oxyde, thiomorpholino S,S-dioxyde, 3-thiazolidinyle S-oxyde ou 3-thiazolidinyle S,S-dioxyde.

Les procédés (a) et (b) ci-dessus sont réalisés par chauffage, à température élevée, pendant plusieurs heures, dans un solvant inerte, de préférence le dioxanne ou l'alcool isopropylique; généralement, ils sont effectués en chauffant à la température d'ébullition du solvant employé et en présence d'une base. La base qui est utilisée pour capter l'acide chlorhydrique qui se dégage au cours de la réaction peut être soit l'amine elle-même mise en oeuvre dans la réaction, soit une autre base organique (par exemple la triethylamine) ou une base minérale (par exemple le carbonate de potassium).

Les composés de départ de formule II sont préparés selon les méthodes conventionnelles, en faisant réagir une 2,4-dichloro-6-R₁-1,3,5-triazine de formule VIII avec la cyclopropylamine de formule V selon l'équation 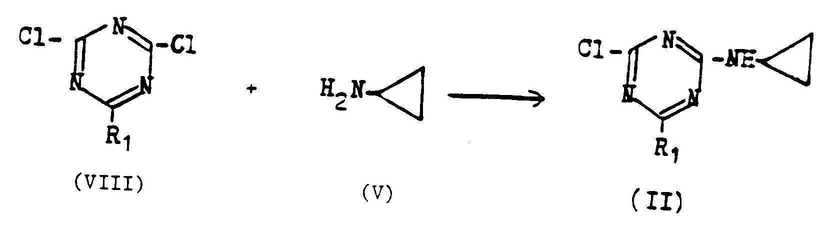 dans ces formules R₁ a la signification donnée ci-dessus.
Cette réaction est généralement effectuée à une température comprise entre -10C° et la température ambiante, dans un solvant inerte tel que le chloroforme et en présence d'une base minérale ou organique, comme par exemple le carbonate de potassium, pour capter l'acide chlorhydrique qui se dégage pendant la réaction.

Quant aux composés de départ de formule IV, ils sont également préparés selon les méthodes conventionnelles, en faisant réagir une 2,4-dichloro-6-R₁-1,3,5-triazine de formule VIII avec une amine de formule R₂H (III), selon l'équation 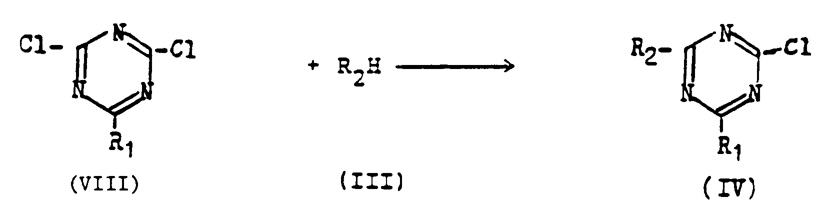 dans ces formules R₁ et R₂ ont les significations données ci-dessus.

Cette réaction est généralement effectuée à une température comprise entre 0°C et 20°C, dans un solvant inerte tel que le chloroforme et en présence d'une base, par exemple le carbonate de potassium.

Les 2,4-dichloro-6-R₁-1,3,5-triazines de formule VIII, utilisées comme produits de départ, peuvent être préparées selon le procédé de R. HIRT et al. (Helv.Chim.Acta, 33,(1950),1365-1369) qui consiste à faire réagir le chlorure de cyanuryle avec un composé organomagnésien approprié de formule R₁MgX dans laquelle R₁ a la signification donnée plus haut et X représente un atome d'halogène, de préférence, un atome d'iode ou de brome.

Les composés de départ de formule VI utilisés dans le procédé (c) sont préparés par un procédé en deux stades :
(1) réaction de la cyclopropylamine de formule V avec le sel de sodium de cyanoguanidine de formule IX pour obtenir la N-cyano-N′-cyclopropylguanidine de formule X, selon l'équation 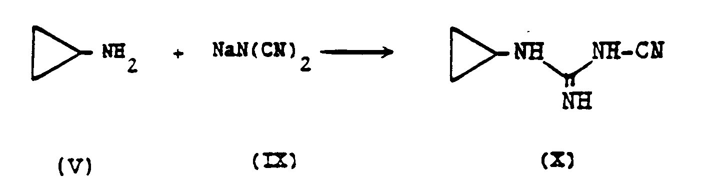
(2) chauffage sous atmosphère inerte pendant plusieurs heures à une température voisine de 160°C de la N-cyano-N′-cyclopropylguanidine de formule X avec une amine de formule R₂H (III) pour obtenir le N-cyclopropylbiguanide de formule VI, selon l'équation 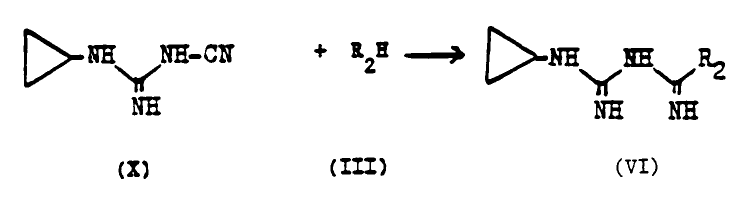 dans ces formules R₂ a la signification donnée ci-dessus.
Quant au procédé (d) par lequel on oxyde une cyclopropylamino-1,3,5-triazine de formule I substituée par un radical thiomorpholino ou 3-thiazolidinyle préparée selon l'un des procédés (a), (b) ou (c), il conduit à la formation du composé S-oxyde ou S,S-dioxyde correspondant selon les conditions opératoires utilisées pour effectuer l'oxydation. Cette oxydation est généralement effectuée au moyen de peroxomonosulfate de potassium (commercialisé sous le nom d'oxone, 2KHS0₅. KHS0₄. K₂S0₄). Lorsque la réaction est conduite à une température comprise entre 10 et 20°C et en présence de 1 à 2 moles d'oxone par mole de composé de formule I à oxyder, on obtient le composé S,S-dioxyde. Par contre, si la température de la réaction est maintenue entre -5°C et +5°C et que l'on utilise seulement environ 0,5 mole d'oxone par mole de composé de formule I, on obtient le composé S-oxyde.

Les sels d'addition d'acides non toxiques pharmaceutiquement acceptables peuvent être préparés à partir des cyclopropylamino-1,3,5-triazines substituées de formule I par des méthodes connues en soi.

Les exemples qui suivent illustrent la présente invention sans la limiter.

### Exemple 1. Préparation des 2,4-dichloro-6-R₁-1,3,5-triazines de départ de formule VIII.

### 2,4-dichloro-6-éthyl-1,3,5-triazine.

A une suspension d'un équivalent de chlorure de cyanuryle dans du toluène, on ajoute goutte à goutte 1,5 équivalent de bromure d'éthylmagnésium en solution dans de l'éther diéthylique (préparé en faisant réagir le magnésium avec du bromure d'éthyle), tout en maintenant la température du mélange réactionnel entre 10 et 15°C. Après l'addition, ce mélange est maintenu à la température ambiante pendant une heure. Ensuite, on ajoute une solution aqueuse contenant 1,5 équivalent d'acide chlorhydrique. On sépare les deux phases, on sèche la phase organique sur du sulfate de sodium, puis on élimine le solvant sous pression réduite. La 2,4-dichloro-6-éthyl-1,3,5-triazine est purifiée par distillation sous pression réduite. Rendement : 63%. P. Eb. : 83°C/17 mbars.

On prépare de la même manière les composés réunis dans le tableau I.

**Tableau I**

| 2,4-dichloro-6-R₁-1,3,5-triazines | | | | |
|---|---|---|---|---|
| R₁ | solvant (1) | solvant (2) | P.Eb.°C/mbars | Rendement (en %) |
| méthyle (3) | Et₂O | toluène | 80-82/16 | 40 |
| n-propyle | Et₂O | toluène | 110/25 | 60 |
| isopropyle | Et₂O | toluène | 87/20 | 29 |
| cyclopropyle | Et₂O | benzène | - (4) | - |
| cyclobutyle | THF | toluène | - (4) | - |
| cyclopentyle | Et₂O | benzène | 135/13 | 25 |

| | | | | |
|---|---|---|---|---|
| Et₂O : éther diéthylique : THF : tétrahydrofuranne. | | | | |
| (1) : solvant utilisé pour préparer le composé organomagnésien, | | | | |
| (2) : solvant aromatique utilisé pour la dispersion du chlorure de cyanuryle, | | | | |
| (3) : composé organomagnésien préparé à partir de l'iodure de méthyle, | | | | |
| (4) : le produit de la réaction n'est pas isolé par distillation : après l'addition du composé organomagnésien, on concentre le mélange réactionnel et on reprend le résidu par de l'éther diéthylique anhydre. On filtre sur Dicalite neutre, on évapore le filtrat et le résidu est utilisé tel quel dans l'étape suivante. | | | | |

### Exemple 2. Préparation des cyclopropylamino-1,3,5-triazines de formule I selon le procédé (a).

A. Obtention des chloro-cyclopropylamino-1,3,5-triazines de départ de formule II.
2-chloro-4-cyclopropylamino-6-méthyl-1,3,5-triazine (composé nouveau).
A une solution molaire de 2,4-dichloro-6-méthyl-1,3,5-triazine dans le chloroforme, préalablement refroidie à -10°C, on ajoute 1 mole de cyclopropylamine en solution dans du chloroforme. Après l'addition, on laisse le mélange revenir à la température ambiante. Ensuite, on refroidit à nouveau le mélange vers 0°C et on y ajoute une solution aqueuse contenant 1 mole de carbonate de potassium. On poursuit l'agitation pendant 1 à 2 heures à la température ambiante. On sépare la phase organique, on la sèche sur sulfate de sodium et on évapore le solvant sous pression réduite. Le résidu est recristallisé dans l'hexane. On obtient ainsi la 2-chloro-4-cyclopropylamino-6-méthyl-1,3,5-triazine. Rendement : 75%. P.F. : 117-119°C.
On prépare de la même manière les composés nouveaux suivants : 2-chloro-4-cyclopropylamino-6-éthyl-1,3,5-triazine.
Le résidu obtenu après évaporation du solvant (rendement brut : 100%) est utilisé tel quel dans l'étape suivante.
2-chloro-4-cyclopropylamino-6-n-propyl-1,3,5-triazine.
Rendement : 100%. P.Eb. : 125°/0,4 mbar.
2-chloro-4-cyclopropylamino-6-isopropyl-1,3,5-triazine.
Ce composé est recristallisé dans l'hexane. Rendement : 74%. P.F. : 79-80°C.
2-chloro-4-cyclopropyl-6-cyclopropylamino-1,3,5-triazine.
Ce composé est recristallisé dans l'hexane. Rendement (calculé à partir du chlorure de cyanuryle) : 71,5%. P.F. : 66-67°C.
2-chloro-4-cyclobutyl-6-cyclopropylamino-1,3,5-triazine.
Rendement brut (calculé à partir du chlorure de cyanuryle) : 23%.
Le produit est utilisé tel quel, sans autre purification, dans l'étape suivante.
2-chloro-4-cyclopentyl-6-cyclopropylamino-1,3,5-triazine.
Rendement (brut) : 100%. Le produit à l'état brut est utilisé tel quel dans l'étape suivante.
B. Obtention des cyclopropylamino-1,3,5-triazines de formule I.
1. 2-cyclopropylamino-4-morpholino-6-n-propyl-1,3,5-triazine (composé 1).
   A une solution contenant 43 g (0,163 mole) de 2-chloro-4-cyclopropylamino-6-n-propyl-1,3,5-triazine dans 300 ml de dioxanne, on ajoute, en maintenant à la température ambiante, 45 ml (0,45 mole) de morpholine dissous dans 200 ml de dioxanne. Après l'addition, on chauffe le mélange à reflux pendant une à deux heures. On laisse ensuite le mélange réactionnel revenir à la température ambiante et on filtre le précipité. On concentre le filtrat et on redissout le résidu dans du dichlorométhane. On lave la solution avec de l'eau. On sépare la phase organique et on la sèche sur sulfate de sodium. Le solvant est évaporé sous pression réduite. Le résidu est purifié par chromatographie sur silice (éluant : 98,5 : 1,5 (v/v) dichlorométhane-éthanol) et le produit est finalement recristallisé dans l'éther diéthylique. On obtient 36,2 g de 2-cyclopropylamino-4-morpholino-6-n-propyl-1,3,5-triazine. Rendement : 85%. P.F. : 104°C.

| Analyse pour C₁₃H₂₁N₅O en % : | | | | | | |
|---|---|---|---|---|---|---|
| calculé | C | 59,29 | H | 8,04 | N | 26,59 |
| trouvé | | 59,20 | | 8,15 | | 26,43 |

On prépare de la même manière les composés réunis dans le tableau II.

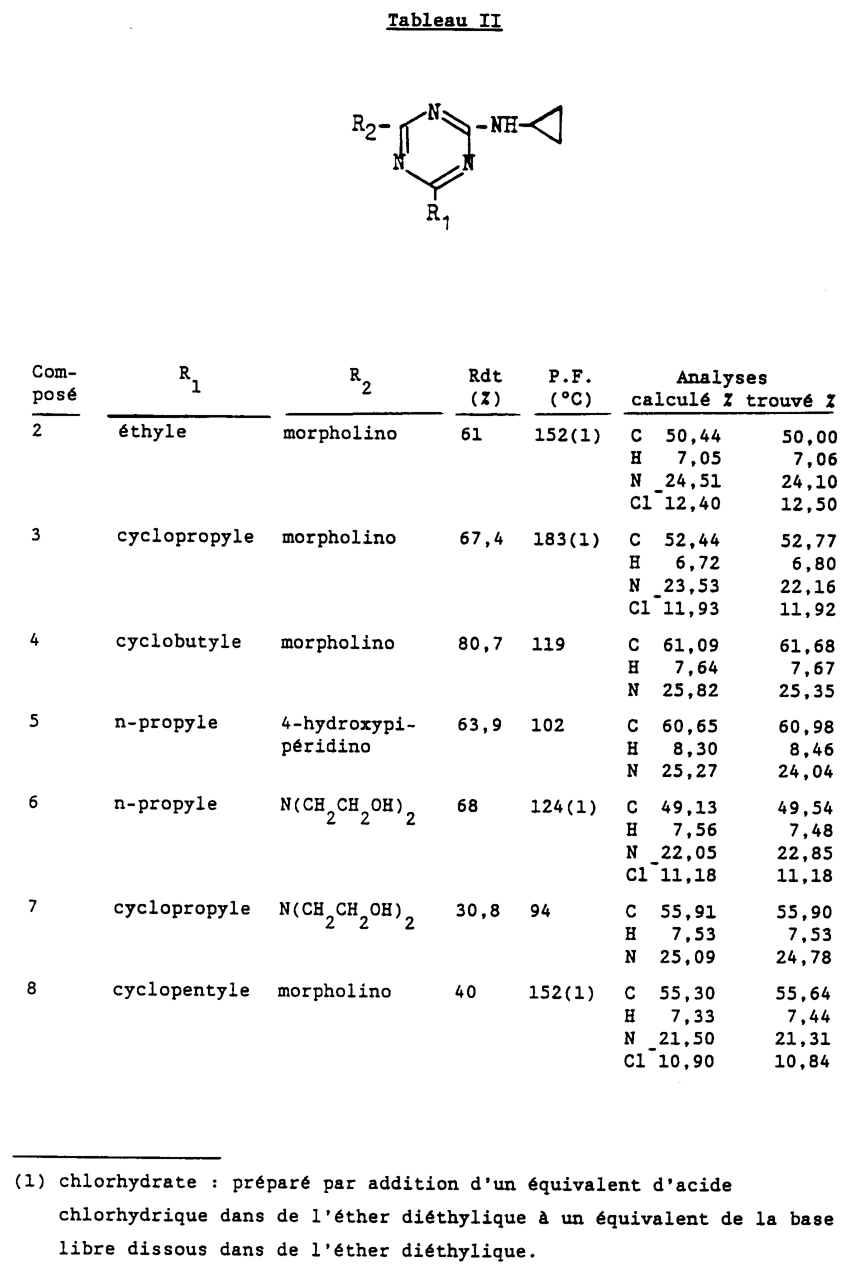

2. 2-cyclopropylamino-4-méthyl-6-(8-oxa-3-azabicyclo[3,2,1]oct-3-yl)-1,3,5-triazine (chlorhydrate) (composé 9). A un équivalent de chlorhydrate de 8-oxa-3-azabicyclo[3,2,1]octane en suspension dans du dioxanne, on ajoute 2 équivalents de triéthylamine en solution dans le même solvant. On ajoute ensuite 1 équivalent de 2-chloro-4-cyclopropylamino-6-méthyl-1,3,5-triazine en solution dans le dioxanne. Le mélange est chauffé à reflux pendant quelques heures. On refroidit à la température ambiante et on filtre le précipité qui s'est formé. On évapore le filtrat sous pression réduite et le résidu est redissous dans du dichlorométhane. On lave la solution avec de l'eau. On sépare la phase organique et on la sèche sur sulfate de sodium, puis on évapore le solvant sous pression réduite. Le résidu ainsi obtenu est purifié par chromatographie sur silice (éluant : 98 : 2 (v/v) dichlorométhane-éthanol). Le chlorhydrate de 2-cyclopropylamino-4-méthyl-6-(8-oxa-3-azabicyclo[3,2,1]oct-3-yl)-1,3,5-triazine est préparé par addition d'un équivalent d'acide chlorhydrique à la base libre dans l'éther diéthylique. Rendement : 63%. P.F. : 220-223°C.

| Analyse pour C₁₃H₁₉N₅O.HCl en % : | | | | | | | | |
|---|---|---|---|---|---|---|---|---|
| calculé | C | 52,44 | H | 6,77 | N | 23,52 | Cl⁻ | 11,92 |
| trouvé | | 52,40 | | 6,74 | | 23,43 | | 11,84 |

Le 8-oxa-3-azabicyclo[3,2,1]octane utilisé comme produit de départ dans cet exemple est un composé connu ; il a été préparé selon la méthode de F.H. NEWS et al. (J. Chem. Soc. 1948, 155-158).

On prépare de la même manière les composés réunis dans le tableau III.

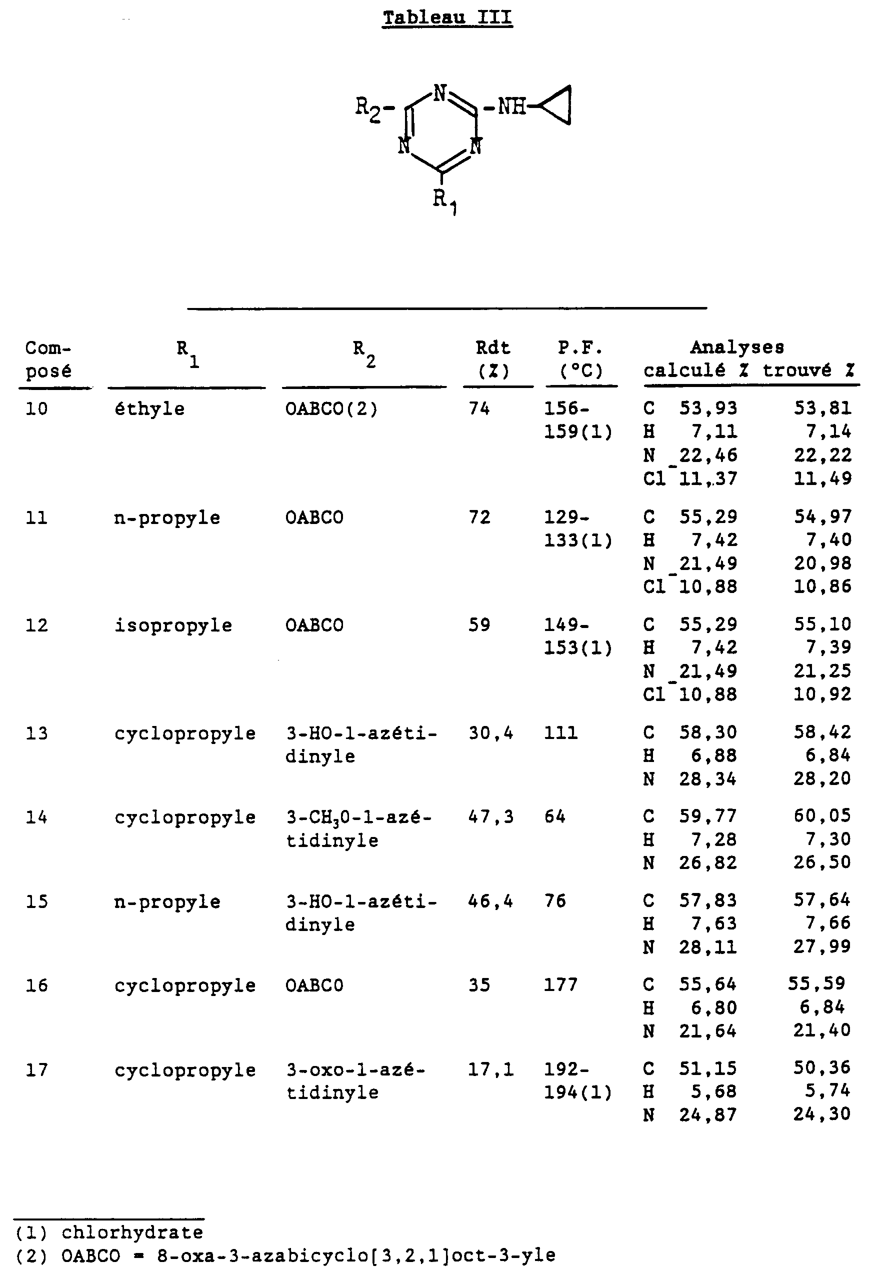

Le chlorhydrate de 3-azétidinone utilisé comme produit de départ pour la synthèse du composé 17 est connu; il a été préparé selon la méthode de H. BAUMANN et al. (Helv. Chim. Acta, 71,(1988),1035).
3.
a) 2-cyclopropyl-4-cyclopropylamino-6-thiomorpholino-1,3,5-triazine (composé 18).
On mélange 12,2 g de 2-chloro-4-cyclopropyl-6-cyclopropylamino-1,3,5-triazine (0,057 mole), 5,97 g de thiomorpholine (0,057 mole) et 8 g de carbonate de potassium (0,057 mole) dans 100 ml d'alcool isopropylique et on chauffe le mélange à 75-80°C pendant 2 heures. On refroidit, on filtre les sels et on évapore le filtrat à sec. On reprend le résidu par 200 ml de dichlorométhane, on lave la solution à l'eau, on la sèche sur sulfate de sodium et on évapore le solvant. On cristallise le produit obtenu dans un mélange 1:2 (v/v) acétate d'éthyle - hezane et on obtient 13,18 g de 2-cyclopropyl-4-cyclopropylamino-6-thiomorpholino-1,3,5-triazine. Rendement : 83,5%. P.F. : 133-134°C.

| Analyse pour C₁₃H₁₉N₅S en % : | | | | | | | | |
|---|---|---|---|---|---|---|---|---|
| calculé | C | 56,32 | H | 6,86 | N | 25,27 | S | 11,55 |
| trouvé | | 56,06 | | 6,93 | | 24,90 | | 11,40 |

On prépare de la même manière les composés suivants :
b) 2-cyclopropyl-4-cyclopropylamino-6-thiomorpholino-1,3,5-triazine S-oxyde (composé 19). Rendement : 23 %. P.F. : 167-168°C.

| Analyse pour C₁₃H₁₉N₅OS en % : | | | | | | | | |
|---|---|---|---|---|---|---|---|---|
| calculé | C | 53,24 | H | 6,48 | N | 23,89 | S | 10,92 |
| trouvé | | 53,10 | | 6,52 | | 23,46 | | 10,62 |

c) 2-cyclopropyl-4-cyclopropylamino-6-thiomorpholino-1,3,5-triazine S,S-dioxyde (composé 20). Rendement : 17%. P.F. : 178-179°C.

| Analyse pour C₁₃H₁₉N₅O₂S en % : | | | | | | | | |
|---|---|---|---|---|---|---|---|---|
| calculé | C | 50,49 | H | 6,15 | N | 22,65 | S | 10,36 |
| trouvé | | 50,72 | | 6,18 | | 22,56 | | 10,35 |

d) 2-cyclopropyl-4-cyclopropylamino-6-(3-thiazolidinyl)-1,3,5-triazine (composé 21). Rendement : 61,2%. P.F. : 110-111°C.

| Analyse pour C₁₂H₁₇N₅S en % : | | | | | | | | |
|---|---|---|---|---|---|---|---|---|
| calculé | C | 54,75 | H | 6,46 | N | 26,61 | S | 12,17 |
| trouvé | | 54,83 | | 6,46 | | 26,68 | | 12,30 |

e) 2-cyclopropyl-4-cyclopropylamino-6-(3-thiazolidinyl)-1,3,5 -triazine S,S-dioxyde (composé 22). Rendement : 35,6%. P.F. : 142-143°C.

| Analyse pour C₁₂H₁₇N₅0₂S en %. | | | | | | | | |
|---|---|---|---|---|---|---|---|---|
| calculé | C | 48,81 | H | 5,76 | N | 23,73 | S | 10,87 |
| trouvé | | 49,11 | | 6,78 | | 23,69 | | 10,96 |

Le chlorhydrate de thiazolidine 1,1-dioxyde utilisé comme produit de départ pour la synthèse du composé 22 ci-dessus est préparé par un procédé en trois stades :
a) N-tert-butoxycarbonyl-thiazolidine.
A une solution de 8,9 g (0,1 mole) de thiazolidine dans 50 ml de dioxanne et 50 ml d'eau, on ajoute goutte à goutte une solution de 24 g (0,11 mole) de dicarbonate de di-tert-butyle dans 50 ml de dioxanne en maintenant le pH entre 10 et 10,5 par addition de lessive de soude. On agite le mélange à la température ambiante pendant 6 heures. On filtre les sels formés et on évapore le solvant organique sous pression réduite. On extrait le résidu aqueux par du dichlorométhane (2 x 50 ml), on décante, on sèche la phase organique sur sulfate de sodium et on évapore le solvant. Le résidu est purifié par distillation sous pression réduite. On obtient 16,3 g de N-tert-butoxycarbonyl-thiazolidine.
Rendement : 86,2%. P.Eb. : 56-57°C/6,7 mbars.
b) N-tert-butoxycarbonyl-thiazolidine 1,1-dioxyde.
On ajoute goutte à goutte à la température ambiante une solution de 30,7 g (0,05 mole) d'oxone (2KHSO₅. KHSO₄. K₂S0₄) dans 300 ml d'eau à une solution de 7,3 g (0,0385 mole) de N-tert-butoxycarbonyl-thiazolidine dans 100 ml de dichlorométhane et 200 ml de méthanol. On agite le mélange à 20°C pendant 24 heures, puis on ajoute 500 ml d'eau, on extrait par du dichlorométhane (3 x 200 ml), on sèche la phase organique sur sulfate de sodium et on évapore le solvant. Le résidu cristallise dans 300 ml d'éther isopropylique. On obtient 6,58 g de N-tert-butoxycarbonyl-thiazolidine 1,1-dioxyde. Rendement : 74,4%. P.F. 106-107°C.

| Analyse pour C₈H₁₅NO₄S en % : | | | | | | | | |
|---|---|---|---|---|---|---|---|---|
| calculé | C | 43,44 | H | 6,79 | N | 6,33 | S | 14,48 |
| trouvé | | 43,52 | | 6,78 | | 6,33 | | 14,36 |

c) Chlorhydrate de thiazolidine 1,1-dioxyde.
On mélange 1,8 g (0,0081 mole) de N-tert-butoxycarbonyl-thiazolidine 1,1-dioxyde et 50 ml d'une solution 2N d'acide chlorhydrique dans de l'éther diéthylique. On agite la suspension à 20°C pendant 6 heures, puis on laisse reposer pendant 48 heures. Le précipité blanc de chlorhydrate de thiazolidine 1,1 -dioxyde est filtré, lavé à l'éther diéthylique et séché. On obtient ainsi 0,83 g de produit. Rendement : 65,3%. P.F. 173-174°C.

| Analyse pour C₃H₇N0₂S. HCl en % : | | | | | | |
|---|---|---|---|---|---|---|
| Calculé | C | 22,86 | H | 5,08 | N | 8,89 |
| trouvé | | 23,29 | | 5,01 | | 8,69 |

### Exemple 3. Préparation des cyclopropylamino-1,3,5-triazines de formule I selon le procédé (b).

A. Obtention des chloro-1,3,5-triazines de départ de formule IV. 2-chloro-4-cyclopropyl-6-morpholino-1,3,5-triazine.
A une solution de 5,7 g (0,03 mole) de 2,4-dichloro-6-cyclopropyl-1,3,5-triazine dans 50 ml de chloroforme, refroidie entre 3 et 5°C, on ajoute en 30 minutes une solution de 2,61 g (0,03 mole) de morpholine dans 20 ml de chloroforme. On laisse la température du mélange remonter jusqu'à environ 10°C, puis on refroidit à nouveau vers 5°C et on ajoute goutte à goutte une solution de 4,14 g (0,03 mode) de carbonate de potassium dans 15 ml d'eau. On agite ensuite le mélange à la température ambiante pendant deux heures. On ajoute 30 ml d'eau et on sépare la phase organique. On lave la solution avec de l'eau, on sèche sur sulfate de sodium et on évapore le solvant sous pression réduite. Le résidu est purifié par chromatographie sur silice (éluant : 96,5:3,5 (v/v) dichlorométhane-acétate d'éthyle) et ensuite recristallisé dans l'hexane. On obtient ainsi 5,5 g de 2-chloro-4-cyclopropyl-6-morpholino-1,3,5-triazine.
Rendement : 76,2%. P.F. : 99-100°C.

| Analyse pour C₁₀H₁₃ClN₄O en % : | | | | | | | | |
|---|---|---|---|---|---|---|---|---|
| calculé | C | 49,89 | H | 5,41 | N | 23,28 | Cl | 14,76 |
| trouvé | | 49,91 | | 5,44 | | 23,06 | | 14,46 |

B. Obtention des cyclopropylamino-1,3,5-triazines de formule I. 2-cyclopropyl-4-cyclopropylamino-6-morpholino-1,3,5-triazine.
A une solution de 5,1 g (0,021 mole) de 2-chloro-4-cyclopropyl-6-morpholino-1,3,5-triazine dans 50 ml de dioxanne, on ajoute à la température ambiante 2,85 g (0,050 mole) de cyclopropylamine en solution dans 20 ml de dioxanne. On chauffe le mélange à reflux pendant 5 heures. Ensuite, on évapore le solvant sous pression réduite et on dissout le résidu dans 50 ml de dichlorométhane et 50 ml d'eau. On sépare la phase organique, on sèche sur sulfate de sodium et on évapore le solvant sous pression réduite. Le résidu cristallise à froid dans l'hexane. On obtient 5,22 g de produit. Ce produit forme un chlorhydrate dans le mélange éthanol-éther diéthylique. On obtient 5,1 g de chlorhydrate de 2-cyclopropyl-4-cyclopropylamino-6-morpholino-1,3,5-triazine, qui est identique au composé 3 préparé à l'exemple 2.B.1. Rendement : 81,7%. P.F. : 183-184°C. (acétonitrile).

| Analyse pour C₁₃H₁₉N₅O.HCl en % : | | | | | | | | |
|---|---|---|---|---|---|---|---|---|
| calculé | C | 52,44 | H | 6,72 | N | 23,53 | Cl⁻ | 11,93 |
| trouvé | | 52,46 | | 6,73 | | 23,44 | | 11,89 |

### Exemple 4. Préparation des cyclopropylamino-1,3,5-triazines de formule I selon le procédé (c).

A. N-cyano-N'-cyclopropylguanidine.
9,36 g (0,1 mole) de chlorhydrate de cyclopropylamine et 8,9 g (0,1 mole) de sel de sodium de cyanoguanidine sont mis en suspension dans 100 ml de n-butanol et 8 ml d'eau. On chauffe le mélange à reflux pendant 2 heures. On filtre la suspension et on lave le gâteau sur le filtre avec 100 ml de n-butanol. Le filtrat est évaporé sous pression réduite. L'huile résiduelle est reprise dans 300 ml d'acétonitrile. On porte à reflux, on filtre à chaud et le gâteau sur le filtre est lavé avec de l'acétonitrile. On évapore le filtrat et l'huile obtenue cristallise lentement. On obtient 7,7 g de N-cyano-N'-cyclopropylguanidine. Rendement : 62%. P.F. : 106°C (alcool isopropylique/éther diéthylique).

| Analyse pour C₅H₈N₄ en % : | | | | | | |
|---|---|---|---|---|---|---|
| calculé | C | 48,37 | H | 6,49 | N | 45,13 |
| trouvé | | 48,40 | | 6,50 | | 45,15 |

B. Obtention des N-cyclopropylbiguanides de départ de formule VI.
1. N-[imino(cyclopropylamino)méthyl]-4-morpholinecarboximidamide (chlorhydrate).
On chauffe un mélange de 4,0 g (32,3 mmoles) de N-cyano-N'-cyclopropylguanidine et de 3,98 g (32,3 mmoles) de chlorhydrate de morpholine à 160°C sous atmosphère d'azote pendant 2 heures et demie. On dissout la masse solide obtenue dans 200 ml d'alcool isopropylique à l'ébullition. On filtre à chaud et le filtrat est concentré jusqu'à apparition d'une suspension solide. On refroidit alors le mélange à la température ordinaire et on ajoute 200 ml d'éther diéthylique. Le produit de la réaction cristallise. On filtre, on lave le produit à l'éther diéthylique et on sèche. On obtient 6,8 g de chlorhydrate de N-[imino(cyclopropylamino)méthyl]-4-morpholinecarboximidamide. Rendement : 85%. P.F. : 195-196°C.

| Analyse pour C₉H₁₇N₅0.HCl en % : | | | | | | | | |
|---|---|---|---|---|---|---|---|---|
| calculé | C | 43,63 | H | 7,32 | N | 28,27 | Cl⁻ | 14,21 |
| trouvé | | 43,60 | | 7,35 | | 27,93 | | 14,18 |

2. N-[imino(cyclopropylamino)méthyl]-4-thiomorpholinecarboximidamide S,S-dioxyde (chlorhydrate).
Ce composé a été préparé comme le composé précédent en faisant réagir le chlorhydrate de thiomorpholine 1,1-dioxyde (brevet britannique 874519) avec la N-cyano-N'-cyclopropylguanidine à 160°C pendant 6 heures. Rendement 58,4%. Le produit obtenu qui est pur à 90% (chromatographie), est utilisé tel quel dans la réaction ultérieure.
C. Obtention des cyclopropylamino-1,3,5-triazines de formule I.
1. 2-cyclopropylamino-4-(1-méthylcyclopropyl)-6-morpholino-1,3,5-triazine. (composé 24).
On dissout 0,48 g (20 mmoles) de sodium dans 20 ml d'éthanol anhydre. On ajoute cette solution à une solution dans l'éthanol contenant 2,48 g (10 mmoles) de chlorhydrate de N-[imino(cyclopropylamino)méthyl]-4-morpholinecarboximidamide, puis on ajoute encore au mélange 2,82 g (22 mmoles) de 1-méthylcyclopropanecarboxylate d'éthyle. Ensuite, on chauffe le mélange à reflux sous azote pendant 88 heures. On refroidit, on évapore l'alcool sous pression réduite et le résidu est redissous dans 50 ml d'eau et 200 ml de dichlorométhane. On sépare la phase aqueuse, on lave deux fois la phase organique avec 50 ml d'eau et on sèche sur sulfate de magnésium. On évapore le solvant sous pression réduite et on recristallise le résidu dans un mélange 1:1 (v/v) éther de pétrole-dichlorométhane. On obtient 0,49 g de 2-cyclopropylamino-4-(1-méthylcyclopropyl)-6-morpholino-1,3,5-triazine. Rendement : 17%. P.F. : 94-95°C.

| Analyse pour C₁₄H₂₁N₅0 en % : | | | | | | |
|---|---|---|---|---|---|---|
| calculé | C | 61,06 | H | 7,69 | N | 25,44 |
| trouvé | | 61,15 | | 7,66 | | 25,56 |

On prépare de la même manière les composés suivants :
2. 2-cyclopropylamino-4-(2-méthylcyclopropyl)-6-morpholino-1,3,5-triazine (chlorhydrate) (composé 25).
Temps de chauffage : 62 heures.
Rendement : 38,7%. P.F. : 177-178°C.

| Analyse pour C₁₄H₂₁N₅0.HCl en % : | | | | | | | | |
|---|---|---|---|---|---|---|---|---|
| calculé | C | 53,93 | H | 7,06 | N | 22,47 | Cl⁻ | 11,40 |
| trouvé | | 54,04 | | 7,14 | | 22,19 | | 11,27 |

3. 2-cyclobutyl-4-cyclopropylamino-6-thiomorpholino-1,3,5-triazine S,S-dioxyde (chlorhydrate) (composé 26).
Temps de chauffage : 18 heures
Rendement : 39%. P.F. : 220-225°C (déc.).

| Analyse pour C₁₄H₂₁N₅0₂S. HCl en % : | | | | | | | | | | |
|---|---|---|---|---|---|---|---|---|---|---|
| calculé | C | 46,73 | H | 6,12 | N | 19,47 | S | 9,87 | Cl⁻ | 8,90 |
| trouvé | | 46,24 | | 6,02 | | 19,14 | | 9,78 | | 8,70 |

4. 2-cyclopropylamino-4-(2-méthylcyclopropyl)-6-thiomorpholino-1,3,5-triazine S,S-dioxyde (composé 27).
Temps de chauffage : 27 heures.
Rendement : 93,9%. P.F. : 180-182°C.

| Analyse pour C₁₄H₂₁N₅0₂S en % : | | | | | | | | |
|---|---|---|---|---|---|---|---|---|
| calculé | C | 52,01 | H | 6,50 | N | 21,67 | S | 9,91 |
| trouvé | | 52,00 | | 6,54 | | 21,36 | | 9,89 |

5. 2-cyclopropylamino-4-(2,2-diméthylcyclopropyl)-6-thiomorpholino-1,3,5-triazine S,S-dioxyde (composé 28).
Temps de chauffage : 6 jours.
Rendement : 40,1 %. P.F. : 170-172°C.

| Analyse pour C₁₅H₂₃N₅0₂S en % : | | | | | | | | |
|---|---|---|---|---|---|---|---|---|
| calculé | C | 53,41 | H | 6,82 | N | 20,77 | S | 9,50 |
| trouvé | | 54,02 | | 6,86 | | 20,88 | | 9,39 |

6. 2-cyclopropylamino-4-(2-n-propylcyclopropyl)-6-thiomorpholino-1,3,5-triazine S,S-dioxyde (composé 29).
Temps de chauffage : 20 heures en autoclave à 110°C.
Rendement : 28,7%. P.F. : 148-149°C.

| Analyse pour C₁₆H₂₅N₅0₂S en % : | | | | | | |
|---|---|---|---|---|---|---|
| calculé | C | 54,70 | H | 7,12 | N | 19,84 |
| trouvé | | 54,71 | | 7,10 | | 20,05 |

### Exemple 5. Préparation des cyclopropylamlno-1,3,5-triazines de formule I selon le procédé (d).

A. 2-cyclopropyl-4-cyclopropylamino-6-(3-thiazolidinyl)-1,3,5-triazine S,S-dioxyde (composé 22).
A une solution de 6,58 g (0,025 mole) de 2-cyclopropyl-4-cyclopropylamino-6-(3-thiazolidinyl)-1,3,5-triazine (composé 21) dans 75 ml de dichlorométhane et 150 ml de méthanol, on ajoute goutte à goutte, à une température comprise entre 10 et 15°C, une solution de 30,7 g (0,05 mole) d'oxone (2KHS0₅. KHS0₄. K₂S0₄) dans 75 ml d'eau. On agite le mélange à la température ambiante pendant 3 heures, puis on ajoute encore 3 g d'oxone dissous dans 10 ml d'eau et l'on maintient l'agitation pendant 2 heures. On ajoute 200 ml d'eau, on extrait par du dichlorométhane (3 x 100 ml), on sépare et on sèche la phase organique sur sulfate de sodium et on évapore le solvant. Le résidu est purifié par chromatographie sur silice (éluant : 98:2 (v/v) dichlorométhane-éthanol). On obtient 3 g de 2-cyclopropyl-4-cyclopropylamino-6-(3-thiazolidinyl)-1,3,5-triazine S,S-dioxyde identique au composé préparé à l'exemple 2.B.3.e.
Rendement : 40,7%. P.F. : 142-143°C (acétate d'éthyle-hexane).
B. 2-cyclopropyl-4-cyclopropylamino-6-(3-thiazolidinyl)-1,3,5-triazine S-oxyde (composé 23).
A une solution de 11,85 g (0,045 mole) de 2-cyclopropyl-4-cyclopropylamino-6-(3-thiazolidinyl)-1,3,5-triazine (composé 21) dans 150 ml de dichlorométhane et 300 ml de méthanol, refroidie vers 0°C, on ajoute goutte à goutte et sans dépasser 5°C, une solution de 15,35 g (0,025 mole) d'oxone dans 150 ml d'eau. Ensuite, on agite encore le mélange à 5°C pendant 30 minutes. On filtre le précipité de sels minéraux sur Hyflo-cel. On élimine la phase organique par décantation, on dilue la phase aqueuse avec 400 ml d'eau et on l'extrait par du dichlorométhane (2 x 200 ml). On sèche la phase organique sur sulfate de sodium et on évapore le solvant. On reprend le résidu d'évaporation dans 100 ml de dichlorométhane, on filtre et on concentre. On obtient un premier jet de 2,83 g de produit solide. La phase aqueuse est alcalinisée et on l'extrait par du dichlorométhane (3 x 100 ml), on sèche la phase organique sur sulfate de sodium et on élimine le solvant. On obtient ainsi 8,41 g de résidu solide (deuxième jet). Les deux jets sont rassemblés et purifiés par chromatographie sur silice (éluant : 93:3 (v/v) dichlorométhane-éthanol). On obtient 7,66 g de 2-cyclopropyl-4-cyclopropylamino-6-(3-thiazolidinyl)-1,3,5-triazine S-oxyde cristallisé dans un mélange acétate d'éthyle-hexane (1:3 v/v).
Rendement : 61%. P.F. 136-137°C.

| Analyse pour C₁₂H₁₇N₅0S en % : | | | | | | | | |
|---|---|---|---|---|---|---|---|---|
| calculé | C | 51,61 | H | 6,09 | N | 25,03 | S | 11,47 |
| trouvé | | 51,79 | | 6,10 | | 25,09 | | 11,50 |

Comme il a été indiqué plus haut, les cyclopropylamino-1,3,5-triazines substituées de formule I, ainsi que leurs sels d'addition d'acides non toxiques pharmaceutiquement acceptables, possèdent la propriété de corriger les effets d'un hypofonctionnement du système cholinergique et ont dès lors une activité favorable sur les processus mnésiques. En outre, ils possèdent une activité sérotonergique centrale et bronchospasmolytique ; ils s'opposent à la libération des médiateurs des mastocytes, ont une activité antiinflammatoire et antioedème et potentialisent l'effet d'un agoniste β-adrénergique sur le relâchement musculaire.

Les essais pharmacologiques décrits ci-après mettent en évidence ces diverses propriétés avantageuses.
1. Activité sur les processus mnésiques.
   Les composés de la présente invention ont été étudiés en vue de faire apparaître d'une part, leur propriété de favoriser l'apprentissage, exprimée par la réduction du nombre d'essais nécessaires pour atteindre un critère prédéterminé, et d'autre part, leur propriété de s'opposer à l'amnésie provoquée par l'administration de scopolamine.
   A cet effet, on a utilisé la méthode de l'évitement passif à essais multiples. Cette méthode est bien connue pour évaluer les effets qu'un produit exerce sur la mémoire et l'apprentissage (A. FINE et al., Proc.Natl.Acad.Sci.USA,82,(1985),5227-5230).
   Le test est pratiqué sur des rats mâles Sprague-Dawley (160-200 g) qui sont maintenus, pendant l'expérience, dans des cages standards. L'appareil utilisé est une cage transparente carrée, de 35 cm de côté et de 25 cm de hauteur, équipée d'un plancher grillagé électrifiable. Un tapis isolant (10 x 17 cm) en caoutchouc est placé sur le sol dans un des coins de la cage.
   Pour évaluer si un composé est susceptible de favoriser l'apprentissage on effectue l'essai suivant.
   Chaque animal est placé sur le tapis en caoutchouc et on note le temps que l'animal met pour se décider à quitter cet emplacement pour explorer la cage. Après 20 secondes d'exploration, l'animal reçoit un choc électrique (durée 3 secondes) dans les pattes, provoquant une réaction de fuite. Le rat est immédiatement retiré de l'appareil et replacé dans sa cage d'origine. On répète cette expérience jusqu'à ce que l'animal reste au moins 180 secondes sur le tapis de caoutchouc pour éviter le choc électrique.
   L'apprentissage est exprimé par le nombre moyen d'essais nécessaires pour atteindre un temps de séjour de 180 secondes sur le tapis.
   Un temps de séjour de 180 secondes sur le tapis de caoutchouc est considéré comme étant la performance maximum à réaliser par l'animal pour éviter le choc électrique. Les rats qui séjournent pendant ce temps sur le tapis ont acquis le réflexe d'évitement et sont replacés dans leur cage d'origine sans recevoir de choc électrique.
   Pour évaluer si un composé est susceptible de favoriser la rétention mnésique au cours du temps on effectue l'expérience suivante. Chaque animal est soumis à quatre essais aux temps 0, 4, 24 et 28 heures.
   Au premier essai (temps 0), l'animal est placé sur le tapis en caoutchouc et on note le temps qu'il met pour se décider à quitter cet emplacement pour explorer la cage. Après 20 secondes d'exploration, le rat reçoit un choc électrique (durée 3 secondes) dans les pattes, provoquant une réaction de fuite. Le rat est immédiatement retiré de l'appareil et replacé dans sa cage d'origine. Au cours des trois essais ultérieurs (temps: 4,24 et 28 heures), l'animal est replacé sur le tapis de caoutchouc et l'on note le temps mis pour quitter cet emplacement. Dès que les quatre pattes de l'animal reposent sur la grille, il reçoit un choc électrique et il est immédiatement retiré de l'appareil.
   Au début de l'expérience, les rats sont répartis en 4 groupes homogènes de 15 animaux. Trente minutes avant chaque essai, chaque groupe d'animaux est soumis à un traitement déterminé:
   - le groupe 1 reçoit une injection intrapéritonéale de sérum physiologique ;
   - le groupe 2 reçoit une injection intrapéritonéale du composé à tester ;
   - le groupe 3 reçoit une injection intrapéritonéale de 0,5 mg de scopolamine et
   - le groupe 4 reçoit une injection intrapéritonéale de 0,5 mg de scopolamine et une injection intrapéritonéale du composé à tester, simultanément.
   Les groupes 1 et 2 sont utilisés dans la première expérience (apprentissage) et les groupes 3 et 4 dans la seconde expérience (rétention mnésique).
   Les résultats obtenus dans ce test avec les composés de l'invention sont repris dans le tableau IV. Dans ce tableau on indique le composé soumis à l'essai (colonne 1) et la dose administrée par voie intrapéritonéale, exprimée en mg/kg (colonne 2).
   Dans les colonnes 3 et 4, on donne les résultats obtenus dans l'essai utilisé pour évaluer l'apprentissage. Les chiffres indiquent le nombre moyen d'essais nécessaires pour qu'un animal témoin (groupe 1) ou traité (groupe 2) par le composé apprenne à séjourner pendant 180 secondes sur le tapis de caoutchouc pour éviter le choc électrique. Les résultats ont été analysés par le test de Student. Dans les colonnes 5 à 12, on donne les résultats obtenus dans l'expérience utilisée pour évaluer la rétention mnésique. Dans les colonnes 5 à 8, les chiffres représentent les temps de séjour moyens observés respectivement aux temps 0, 4, 24 et 28 heures pour les animaux du groupe 3, traités seulement à la scopolamine, et dans les colonnes 9 à 12, on trouve les chiffres correspondants pour les animaux du groupe 4, traités simultanément par la scopolamine et par le composé étudié (à la dose indiquée dans la deuxième colonne).
   L'influence favorable d'un composé pour s'opposer à l'amnésie provoquée par la scopolamine est démontrée par l'augmentation du temps de séjour sur le tapis, à chaque observation. Les différences observées sont analysées statistiquement par la méthode de Mann-Whitney.

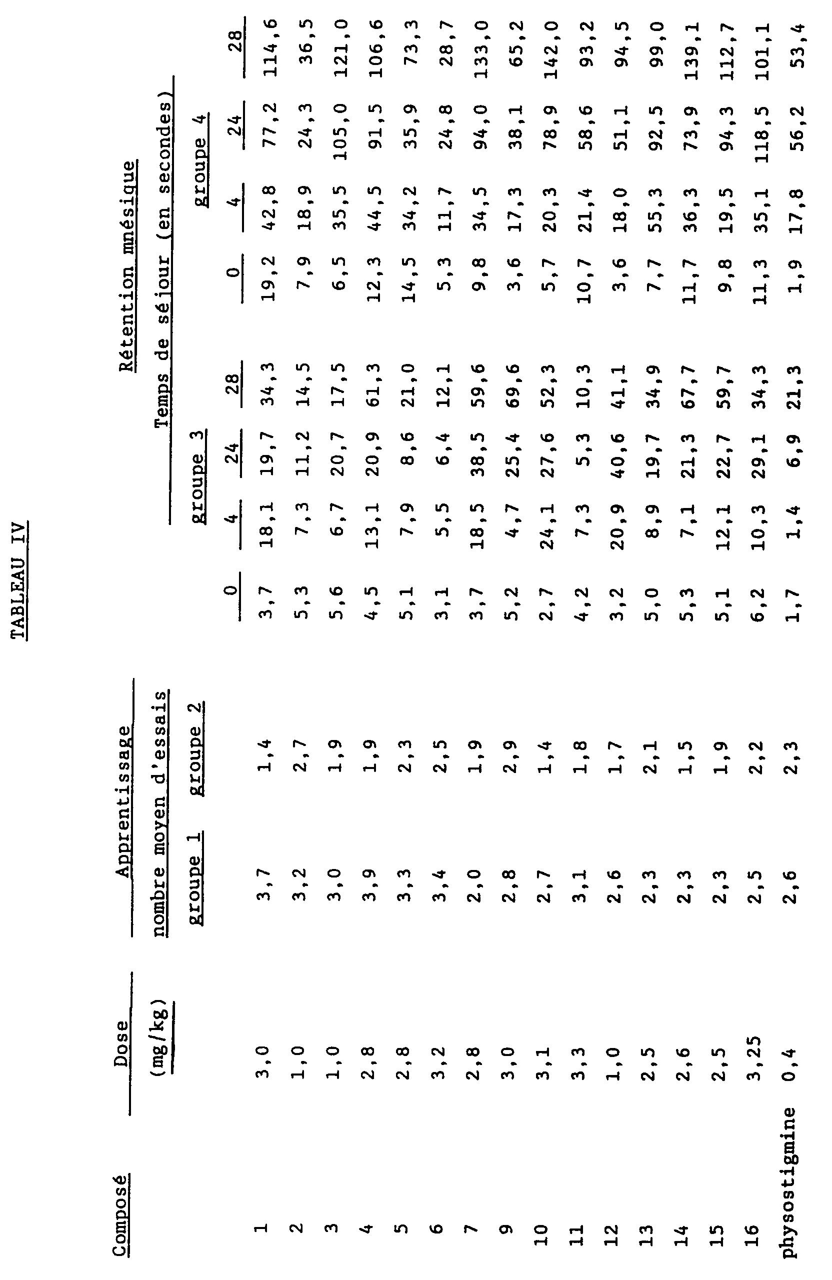

De ce tableau, il apparaît que :
- les composés de l'invention favorisent l'apprentissage du réflexe d'évitement: le nombre moyen d'essais nécessaires pour atteindre le critère prédéterminé (temps de séjour maximum de 180 secondes sur le tapis) est moins élevé pour les animaux traités (colonne 4) que pour les animaux témoins (colonne 3);
- l'effet amnésiant de la scopolamine est très marqué: on voit que les temps de séjour des animaux du groupe 3 (colonnes 5 à 8) sont nettement moindres que les 180 secondes réalisées par les témoins après un nombre moyen d'essais (colonne 3); et, dans ces conditions, l'influence favorable des composés de l'invention pour s'opposer à l'effet amnésiant de la scopolamine est très nette: les animaux du groupe 4, traités simultanément par la scopolamine et par un composé de l'invention, ont des temps de séjour, à chaque observation, considérablement plus élevés que les animaux du groupe 3 traités à la scopolamine seule (comparer les résultats de la colonne 5 avec ceux de la colonne 9, 6 avec 10, etc.).
- la physostigmine exerce un effet favorable contre l'effet amnésiant de la scopolamine, similaire à celui des composés de l'invention mais à une dose présentant des effets secondaires et très proche de la dose toxique, ce qui n'est pas le cas pour les composés de l'invention.

2. Activité sérotonergique.
Une diminution de l'activité sérotonergique a été mise en corrélation avec l'apparition de troubles affectifs comme la dépression et l'anxiété. L'injection à des rats de sérotonine ou de 5-hydroxytryptophane (5-HTP), un agoniste de la sérotonine, induit chez cet animal des secousses paroxystiques de la tête, du cou et du tronc qui rappellent les frémissements d'un chien trempé qui s'ébroue. Ce comportement est appelé "wet dog shake" (WDS) et est utilisé comme modèle pour mettre en évidence les propriétés aminergiques et en particulier sérotonergiques que l'on retrouve chez les antidépressants (P. BEDARD et C.J. PYCOCK, Neuropharmacology, 16,(1977),663-670).
L'essai est pratiqué le matin sur des rats mâles Sprague-Dawley (± 180 g) répartis la veille par groupes de 8 animaux dans des cages de séjour.
La cage utilisée pour les essais est une enceinte transparente de 12 x 24 x 30 cm de hauteur, dont le sol est recouvert d'une couche de sable.
Les composés à tester, mis en solution soit dans du sérum physiologique, soit dans un tampon citrate à pH5, sont administrés par voie intrapéritonéale, à une dose différente pour chaque groupe traité. Les groupes témoins reçoivent une injection intrapéritonéale du même véhicule (soit sérum physiologique, soit tampon citrate).
Après l'administration du produit, les animaux sont placés immédiatement dans la cage d'essai par groupe de quatre à la fois, et après une période d'habituation d'une durée de 10 minutes, on compte le nombre de secousses (WDS) qui se produisent pendant 30 minutes.
Les valeurs moyennes des résultats sont calculées et analysées statistiquement par la méthode de Mann-Whitney.
Dans le tableau V ci-dessous, on donne les valeurs moyennes du nombre de secousses obtenu pour les composés de l'invention administrés par voie intrapéritonéale aux doses indiquées (en mg/kg).

**Tableau V**

| Comportement "Wet Dog Shake" | | |
|---|---|---|
| Composé | Dose (mg/kg) | Moyenne du nombre de secousses |
| 1 | 1,0 | 7,8 ± 1,6 |
| 2 | 1,0 | 10,0 ± 3,5 |
| 3 | 0,3 | 5,1 ± 1,0 |
| | 1,0 | 11,9 ± 2,7 |
| 4 | 2,8 | 10,6 ± 4,3 |
| 5 | 2,8 | 8,4 ± 1,6 |
| 6 | 1,0 | 1,9 ± 0,5 |
| | 3,2 | 3,6 ± 1,1 |
| 7 | 2,8 | 7,9 ± 1,8 |
| 10 | 3,1 | 16,3 ± 3,8 |
| 11 | 1,1 | 4,3 ± 1,4 |
| 12 | 3,2 | 16,3 ± 4,2 |
| 13 | 2,5 | 5,6 ± 1,8 |
| 14 | 0,8 | 2,1 ± 0,7 |
| 16 | 1,0 | 10,6 ± 1,8 |
| 17 | 2,8 | 10,5 ± 2,9 |
| 18 | 0,88 | 10,6 ± 3,0 |
| 19 | 0,94 | 16,0 ± 4,7 |
| 20 | 0,99 | 13,6 ± 2,3 |
| 21 | 2,63 | 5,5 ± 2,0 |
| 22 | 0,95 | 8,1 ± 1,7 |
| 23 | 0,89 | 10,1 ± 3,0 |
| 24 | 2,75 | 6,1 ± 2,3 |
| 25 | 1,0 | 19,6 ± 5,0 |
| 26 | 1,15 | 11,8 ± 4,3 |
| 5-HTP/carbidopa(1) | 100,0 | 4,7 ± 1,5 |
| | 200,0 | 19,6 ± 3,0 |

| | | |
|---|---|---|
| (1) les animaux sont prétraités par l'inhibiteur de la décarboxylase périphérique, l'α-méthyldopahydrazine ou carbidopa. (25 mg/kg, voie ip., 30 minutes avant le 5-HTP) ; les mesures sont effectuées 90 à 120 minutes après l'injection intrapéritonéale de 5-HTP. | | |

Ce tableau montre que les composés de l'invention induisent chez le rat un comportement "wet dog shake" comparable à celui provoqué par des injections d'un agoniste sérotonergique tel que le 5-HTP en présence de carbidopa, mais à des doses nettement plus faibles.
3. Activité bronchospasmolytique.
Cette activité a été mesurée chez le cobaye Dunkin-Hartley par la méthode de H. KONZETT et R. ROESSLER (Naunyn Schmiedebergs Arch.exp.Path. Pharmacol.,195,(1940),71-74) et comparée à celle de la théophylline.
Le cobaye anesthésié (uréthane) et curarisé (gallamine) est placé sous respiration assistée. La pression endotrachéale est enregistrée. Des spasmes bronchiques répétés sont induits par des injections intraveineuses successives (toutes les 5 minutes) respectivement de sérotonine, d'histamine ou d'acétylcholine à une dose capable de provoquer une augmentation de la pression endotrachéale de 20 à 50 cm d'eau.
Le composé à tester est également administré par voie intraveineuse deux minutes avant l'administration du spasmogène et ensuite en 3 à 4 doses cumulatives en quantités croissantes, à 15 minutes d'intervalle. On utilise 6 animaux par composé à tester et par spasmogène.
Dans le tableau VI ci-dessous figurent les doses de produits (DI50 en µmol/kg) qui inhibent de 50%, en moyenne sur l'ensemble des animaux, les bronchospasmes induits.

**Tableau VI**

| Activité bronchospasmolytique | | | |
|---|---|---|---|
| Composé | Doses (DI50 en µmol/kg) | | |
| | Sérotonine | Histamine | Acétylcholine |
| 1 | 0,01 | 0,01 | 0,1 |
| 2 | 0,5 | 0,3 | 0,5 |
| 3 | 0,1 | 0,03 | 0,07 |
| 4 | 0,03 | 0,004 | 0,03 |
| 5 | 0,1 | 0,06 | 0,1 |
| 6 | 0,1 | 0,1 | 0,3 |
| 7 | 0,4 | 0,2 | 0,7 |
| 8 | 0,3 | 0,1 | 0,2 |
| 9 | 1,0 | 0,4 | 2,1 |
| 10 | 0,3 | 0,1 | 0,5 |
| 11 | 0,1 | 0,1 | 0,09 |
| 12 | 0,06 | 0,04 | 0,2 |
| 13 | 0,6 | 0,1 | 1,3 |
| 14 | 0,2 | 0,09 | 0,5 |
| 15 | 0,2 | 0,08 | 0,3 |
| 16 | 0,009 | 0,009 | 0,05 |
| 17 | 0,2 | 0,1 | 0,2 |
| 18 | - | 0,003 | - |
| 19 | 0,04 | 0,02 | 0,1 |
| 20 | 0,4 | 0,03 | 0,1 |
| 24 | - | 0,04 | - |
| 26 | - | 0,03 | - |
| théophylline | 4,6 | 5,6 | 10,0 |

Il ressort de ce tableau que les composés de l'invention ont une activité bronchospasmolytique remarquable à l'égard des bronchospasmes induits respectivement par la sérotonine, l'histamine ou par l'acétylcholine.
4. Activité antiinflammatoire et antioedème.
La réaction entre un antigène soluble et des anticorps dans l'organisme peut conduire à une réaction inflammatoire aiguë accompagnée de libération d'histamine, de modification de la perméabilité vasculaire et de la formation d'un oedème localisé.
Le test "Arthus passif inverse" (RPA) a pour objectif de mettre en évidence les propriétés antiinflammatoires d'un composé sur l'oedème plantaire induit expérimentalement par des complexes immuns chez le rat Sprague-Dawley (P.J. BAILEY et A. STURM, Biochem. Pharmacol., 32, (1983), 475).
A cet effet, on provoque la réaction d'Arthus par l'administration subplantaire de 0,1 ml de sérum réaginique antiovalbumine hétérologue dans la patte arrière droite et par l'injection intraveineuse simultanée de 1 ml/kg d'ovalbumine (25 mg/ml).
Le composé à tester est administré par voie intraveineuse 30 secondes avant l'induction de la réaction d'Arthus, au moins à 3 doses différentes. On utilise un groupe de 6 rats par dose de composé à tester.
Le volume de la patte est mesuré par pléthysmométrie avant la réaction d'Arthus et 3 à 5 heures après l'induction de la réaction d'Arthus, aussi bien chez les animaux témoins que chez les animaux traités.
L'effet d'un composé sur la diminution de l'oedème, pour chaque dose et à chaque temps de mesure (3 heures et 5 heures) est exprimé en % de l'oedème observé chez les animaux témoins.
Dans le tableau VII ci-dessous, on donne les doses de produits (DI30 en µmoles/kg) qui inhibent de 30%, en moyenne sur l'ensemble des animaux, le volume de l'oedème observé chez les animaux témoins.

**Tableau VII**

| Activité antiinflammatoire et antioedème | | |
|---|---|---|
| Composé | Dose (DI30 en µmoles/kg) | |
| | 3 heures | 5 heures |
| 1 | 2,0 | 2,0 |
| 2 | 2,3 | 0,7 |
| 3 | 0,8 | 0,4 |
| 4 | 9,0 | 10,0 |
| 5 | 3,1 | 27,0 |
| 6 | 12,0 | 9,0 |
| 7 | 7,7 | 11,0 |
| 8 | 4,0 | 4,0 |
| 9 | 3,4 | 4,4 |
| 11 | 0,5 | 0,4 |
| 12 | 2,5 | 3,0 |
| 13 | 9,0 | 4,0 |
| 14 | 16,0 | 12,0 |
| 15 | 39,0 | 4,0 |
| 16 | 1,5 | 2,0 |
| 17 | 1,0 | 1,0 |
| 18 | 1,0 | 1,0 |
| 19 | 0,02 | 0,02 |
| 20 | 1,0 | 0,5 |
| théophylline | 18,0 | 10,0 |

Il ressort de ce tableau que les composés de l'invention possèdent une activité antiinflammatoire et antioedème nettement supérieures à celles de la théophylline.
5. Inhibition de la libération anaphylactique d'histamine.
Ce test a un double objectif : d'une part, évaluer "in vitro" l'activité inhibitrice d'une substance sur la libération anaphylactique de l'histamine provoquée par la dégranulation des mastocytes de poumons de cobayes, et d'autre part, mettre en évidence un éventuel effet de potentialisation (synergie) de l'activité inhibitrice d'un agoniste β-adrénergique, par exemple l'isoprénaline, sur cette même libération d'histamine. (E.S.K. ASSEM et H.O. SCHILD, Int. Arch. Allergy, 40, (1971), 576-589).
On utilise des cobayes Dunkin-Hartley qui sont tout d'abord sensibilisés passivement par une injection intraveineuse de 1 ml de sérum isologue antiovalbumine. Ensuite, vingt-quatre heures après l'injection, les poumons sont perfusés par un tampon Tyrode pour évacuer le sang, puis prélevés et découpés en tranches de 1 mm. Ces tranches sont réparties dans des tubes à essai (3 tranches par tube) de manière à obtenir 10 groupes expérimentaux de tranches de poumons par cobaye.
Un groupe témoin "positif" de tranches de poumons est stimulé par addition d'une solution d'ovalbumine (0,1 mg/ml) pour déclencher la libération anaphylactique de l'histamine et sert à déterminer la quantité maximale d'histamine (100%) qui peut être libérée.
Un groupe témoin "négatif", non stimulé par l'ovalbumine, sert à déterminer la quantité d'histamine libérée naturellement (libération spontanée).
Dans trois autres groupes, les tranches de poumon sont incubées à 37°C pendant 30 minutes dans la solution d'ovalbumine en présence de trois doses différentes du composé à tester (une dose par groupe).
Pour déceler un effet de potentialisation d'un composé de l'invention sur l'inhibition par l'isoprénaline de la libération de l'histamine, trois autres groupes de tranches de poumon sont traités de la même manière que ci-dessus, mais le milieu d'incubation contient en outre de l'isoprénaline à la dose de 10⁻⁷ mole par litre. A cette dose, l'isoprénaline inhibe de 30 à 40% la libération d'histamine, ce qui est vérifié sur un groupe témoin qui n'est traité que par de l'isoprénaline seule à cette dose.
En outre, un groupe témoin supplémentaire, non stimulé par l'ovalbumine, est utilisé pour détecter une éventuelle libération spontanée d'histamine par la plus forte dose du composé étudié.
Pour chaque groupe, l'histamine libérée dans le milieu d'incubation est dosée par spectrofluorométrie (D.P. EVANS et al., Life Sci., 12, (1973), 327-336).
Les résultats obtenus permettent de déterminer pour chaque composé, une dose minimale inhibitrice (DMI) (effet propre), c'est-à-dire la dose de composé pour laquelle la quantité d'histamine libérée est plus faible que celle du groupe témoin "positif" et pour laquelle cette différence est statistiquement significative, et une dose minimale potentialisatrice (DMP) qui est la dose de composé qui induit une inhibition supérieure à celle de l'isoprénaline à la dose de 10⁻⁷ mole par litre.
Dans le tableau VIII ci-dessous, on donne les doses minimales inhibitrices (DMI) et les doses minimales potentialisatrices (DMP), exprimées en µmole/l, obtenues à ce test avec les composés de l'invention et avec la théophylline comme substance de référence.

**Tableau VIII**

| Inhibition de la libération anaphylactique d'histamine | | |
|---|---|---|
| Composé | Dose minimale inhibitrice (en µmole/1) | Dose minimale potentialisatrice (en µmole/1) |
| 1 | 0,1 | 0,32 |
| 2 | 1,0 | 3,2 |
| 3 | 0,032 | 0,32 |
| 4 | 0,32 | 0,1 |
| 5 | 0,32 | 3,2 |
| 6 | 1,0 | 1,0 |
| 7 | 0,32 | 1,0 |
| 8 | 1,0 | 10,0 |
| 9 | 3,2 | 3,2 |
| 10 | 3,2 | 0,32 |
| 11 | 1,0 | 0,32 |
| 12 | 0,1 | 0,1 |
| 13 | 1,0 | 1,0 |
| 14 | 1,0 | 0,32 |
| 15 | 10,0 | 3,2 |
| 16 | 0,1 | 0,032 |
| 17 | 3,2 | 1,0 |
| 18 | 0,1 | 1,0 |
| 19 | 1,0 | 0,1 |
| 20 | 1,0 | 0,32 |
| 21 | 0,1 | 0,32 |
| 22 | 0,1 | 0,032 |
| 24 | 0,032 | 0,32 |
| 25 | 0,032 | 0,032 |
| 26 | 1 | 0,32 |
| théophylline | 100,0 | 1000,0 |

Il ressort de ce tableau que les composés de l'invention sont beaucoup plus actifs que la théophylline pour inhiber la libération anaphylactique de l'histamine (effet propre) et possèdent en même temps, à des doses faibles, un effet de potentialisation de l'effet d'inhibition de l'isoprénaline, obtenu à la dose de 10⁻⁷ mole par litre.
6. Potentialisation de l'effet myorelaxant de l'isoprénaline sur l'iléon de cobaye.
(cf. R.A. TURNER, "Screening Methods in Pharmacology", Ed. Acad. Press, San Diego, 1965, chapitre IV, pages 43-47).
Des fragments de muscles longitudinaux d'iléon de cobayes Dunkin-Hartley (250 à 500 g), attachés à un indicateur de force isométrique, sont plongés dans une solution de Tyrode (37 ± 1°C, pH 7,6 ; 5,6 mmoles/l de glucose) oxygénée par le passage d'un courant de gaz (mélange 95% O₂ - 5% CO₂) et sont tendus avec une force de 1 g. Après stabilisation de la tension, des cycles successifs d'injection d'histamine (dichlorhydrate d'histamine à 3,2 µmoles/l) sont effectués au moyen d'une pompe à perfusion de type Braun.
Chaque cycle d'injection comprend les 6 phases successives suivantes : une phase de repos (durée : 25 secondes), une phase d'injection d'histamine (durée : 6 secondes), une période de contraction musculaire (durée : 24 secondes), un premier lavage à l'eau (durée : 25 secondes), une période de stabilisation pendant laquelle le muscle revient à la tension de départ (durée : 35 secondes), suivie d'un second lavage à l'eau (durée : 25 secondes).
Cycle d'inhibition : lorsque les contractions successives induites par les injections d'histamine sont devenues reproductibles, le composé à tester est injecté immédiatement après le second lavage. La contraction induite par l'injection suivante d'histamine est mesurée et comparée à la moyenne des trois contractions précédentes provoquées par les injections répétées d'histamine (contractions témoins). A partir du résultat obtenu, on calcule le pourcentage d'inhibition de la contraction. Ensuite, plusieurs cycles d'injection d'histamine sont à nouveau effectués jusqu'à ce que les contractions du muscle redeviennent reproductibles; le muscle est alors prêt pour tester un autre composé.
L'effet de potentialisation d'un composé est mesuré au cours d'un cycle dit "potentialisation" qui comprend trois cycles d'inhibition successifs. Dans le premier cycle d'inhibition, on détermine le pourcentage d'inhibition de la contraction obtenu par l'injection d'isoprénaline seule à la dose de 10⁻⁷ mole/l. A cette dose, le pourcentage d'inhibition de la contraction est habituellement compris entre 10 et 25%. Au cours du deuxième cycle d'inhibition, on détermine le pourcentage d'inhibition de la contraction obtenu avec le composé à tester, injecté seul à une dose donnée. Dans le troisième cycle d'inhibition, l'isoprénaline et le composé à tester sont injectés simultanément, et le pourcentage d'inhibition de la contraction est calculé pour la dose utilisée du composé étudié. Les mêmes expériences sont répétées pour chacune des doses des composés étudiés.
A partir des résultats obtenus on détermine pour chaque composé testé, la dose minimale potentialisatrice (DMP) qui correspond à la dose de produit pour laquelle l'inhibition obtenue avec le mélange composé + isoprénaline est significativement supérieure (p<0,05) à la somme de chacune des inhibitions obtenues lorsque le composé et l'isoprénaline sont injectés isolément.
Dans le tableau IX ci-dessous, on donne pour les composés de l'invention, comparés à la théophylline, la dose minimale exprimée en µmole/l, qui potentialise l'effet myorelaxant de l'isoprénaline administrée à la dose de 10⁻⁷ mole/l.

**Tableau IX**

| Potentialisation de l'effet myorelaxant de l'isoprénaline sur l'iléon de cobaye. | |
|---|---|
| Composé | Dose minimale potentialisatrice (en µmole/l) |
| 1 | 0,032 |
| 2 | 1,0 |
| 3 | 0,032 |
| 4 | 0,032 |
| 5 | 1,0 |
| 6 | 1,0 |
| 7 | 0,32 |
| 9 | 10,0 |
| 10 | 1,0 |
| 11 | 0,032 |
| 12 | 0,01 |
| 13 | 1,0 |
| 14 | 1,0 |
| 15 | 3,2 |
| 16 | 0,032 |
| 17 | 0,32 |
| 18 | 0,1 |
| 19 | 0,32 |
| 20 | 0,1 |
| théophylline | 1000,0 |

Ce tableau montre que les composés de l'invention sont beaucoup plus actifs que la théophylline pour potentialiser l'effet myorelaxant de l'isoprénaline.
7. Effet sur les granulocytes polymorphonucléaires neutrophiles .
Les granulocytes polymorphonucléaires neutrophiles (PMN) sont des cellules qui sont mobilisées dans les phénomènes inflammatoires et qui peuvent être stimulées par diverses substances, telles que par exemple la formylméthionyl-leucyl-phénylalanine (FMLP) ou les prostaglandines E (PGE₁). Les granulocytes PMN répondent à ces stimuli extracellulaires par une activation du métabolisme de l'oxygène avec libération de métabolites oxygénés toxiques. Une réponse excessive des granulocytes PMN peut être la cause d'une inflammation douloureuse et s'accompagne également d'une diminution du taux d'adénosine monophosphate cyclique (cAMP) dans ces granulocytes. Il en résulte que des composés qui inhibent la poussée respiratoire des granulocytes PMN ou qui augmentent le taux de cAMP peuvent être considérés comme très importants pour le traitement de l'arthrite et de l'asthme. L'essai pharmacologique décrit ci-dessous a pour objectif de montrer que les composés de l'invention présentent un double caractère : d'une part, ils inhibent la stimulation des granulocytes PMN et, d'autre part, ils augmentent le taux de cAMP dans ces cellules.
- Inhibition de la stimulation des granulocytes PMN.
La stimulation des granulocytes PMN est mise en évidence par la chimiluminescence qui accompagne l'activation du métabolisme de l'oxygène lorsque ces cellules sont stimulées en présence de luminol (5-amino-2,3-dihydro-1,4-phtalazinedione).
Des granulocytes PMN de rat (5 x 10⁶/ml) sont préincubés dans un tampon phosphate (150 µmoles/litre, pH 7,4) contenant du luminol à la concentration de 10⁻⁵ mole par litre, pendant 15 minutes à 37°C, puis pendant 5 minutes en présence du composé à tester à la concentration de 10⁻⁶ mole/litre.
La réaction de stimulation des granulocytes PMN est initiée par l'addition dans le milieu de FMLP à la concentration finale de 3,2 x 10⁻⁷ mole/litre. La luminescence qui résulte de la stimulation est mesurée au moyen d'un luminomètre LKB 1251 suivant la méthode de C. DAHLGREN et O. STENDAHL (Infection and Immunology, 37, (1982), 34 à 39). Un cycle expérimental dure 38 secondes. La réaction est répétée 9 fois pour chaque composé étudié et l'on calcule la moyenne des résultats obtenus.
Dans le tableau X ci-dessous, on donne pour les composés de l'invention et pour la théophylline (composé de référence), le pourcentage moyen de la chimiluminescence résiduelle calculé par rapport à un essai témoin au cours duquel les granulocytes PMN sont incubés et stimulés par la FMLP en l'absence de composé à tester (100% de chimiluminescence).

**Tableau X**

| Inhibition de la stimulation des granulocytes PMN. | |
|---|---|
| Composé (10⁻⁶ mole/l) | Chimiluminescence résiduelle (en %) |
| 1 | 47 |
| 2 | 68 |
| 3 | 39 |
| 4 | 45 |
| 5 | 74 |
| 6 | 55 |
| 7 | 54 |
| 9 | 82 |
| 10 | 56 |
| 11 | 44 |
| 12 | 36 |
| 13 | 79 |
| 14 | 51 |
| 15 | 70 |
| 16 | 42 |
| théophylline | 100 |

Il ressort de ce tableau qu'à la concentration de 10⁻⁶ mole/l, concentration à laquelle tous les composés de l'invention sont testés, la théophylline est complètement inactive. Par contre, on voit qu'à cette concentration, les composés de l'invention provoquent une diminution importante de la chimiluminescence et induisent donc une inhibition importante de la stimulation des granulocytes PMN.
Il a été observé en outre qu'il faut une concentration 100 fois plus élevée (10⁻⁴ mole/l) pour obtenir un effet comparable avec la théophylline (chimiluminescence résiduelle de 65%).
- Augmentation du taux de cAMP.
Des granulocytes PMN de rat (10⁷ dans 200 µl) sont incubés dans un tampon phosphate (150 µmoles/litre, pH 7,4) à 37°C pendant 3 minutes en présence du composé à tester à la concentration de 3,2.10⁻⁶ mole par litre et de PGE₁ à la concentration de 10⁻⁶ mole/litre. La réaction est ensuite arrêtée par l'addition de 1 ml de propanol. Après centrifugation à 15.000 g pendant 3 minutes, le surnageant est récupéré, évaporé, et la quantité de cAMP dans le résidu est déterminée par un dosage radioimmunologique selon le procédé recommandé par le fournisseur du réactif utilisé à cet effet (Amersham).
Un essai témoin est effectué dans les mêmes conditions, mais en l'absence de composé à tester.
Dans le tableau XI ci-dessous, on donne les quantités de cAMP (exprimées en picomoles) obtenues au cours de ces essais par comparaison avec la théophylline, utilisée également à la concentration de 3,2.10⁻⁶ mole/litre.

**Tableau XI**

| Composé | Quantité de cAMP produite (en picomoles pour 10⁷ cellules) |
|---|---|
| 1 | 21,0 |
| 3 | 23,2 |
| 4 | 17,7 |
| 5 | 11,6 |
| 6 | 14,6 |
| 7 | 15,8 |
| 11 | 16,2 |
| 12 | 16,8 |
| théophylline | 5,0 |
| témoin | 4,3 |

Ce tableau montre que les composés de l'invention sont plus actifs que la théophylline et augmentent considérablement le taux de cAMP dans les granulocytes PMN stimulés par PGE₁.
8. Toxicité.
La toxicité des composés de l'invention a été déterminée chez la souris mâle NMRI au moyen du test d'Irwin (S. IRWIN, Psychopharmacologia, 13, (1968), 222-257).
Des doses progressives du composé à tester sont administrées par voie intrapéritonéale à des groupes de trois souris jusqu'à ce que la dose mortelle soit atteinte (dose provoquant la mort de deux animaux sur trois dans les 48 heures).
Dans le tableau XII ci-dessous, on donne la dose mortelle observée pour les composés de l'invention. Il ressort de ce tableau que les composés de l'invention sont très peu toxiques, contrairement à la physostigmine.

**Tableau XII**

| Composé | Dose mortelle (en mg/kg) |
|---|---|
| 1 | 898 |
| 2 | 285 |
| 3 | 297,8 |
| 4 | 165 |
| 5 | 277,4 |
| 6 | 635,6 |
| 7 | 279,3 |
| 8 | 326 |
| 9 | 297,8 |
| 10 | 311,8 |
| 11 | 325,8 |
| 12 | 325,8 |
| 13 | 247,3 |
| 14 | 156,8 |
| 15 | >498 |
| 16 | 194 |
| 17 | 281,5 |
| 18 | 277 |
| 19 | 175,6 |
| 20 | 185 |
| 21 | 263 |
| 22 | 295 |
| 23 | 156 |
| 24 | 825 |
| 25 | 99,7 |
| 26 | 201,5 |
| physostigmine | O,82 |

9. Posologie et administration.
Les compositions pharmaceutiques renfermant les composés de l'invention peuvent être administrées par voie orale, parentérale ou rectale. Les compositions pharmaceutiques utilisables pour l'administration orale peuvent être solides ou liquides, par exemple sous forme de comprimés (enrobés ou non), pilules, dragées, capsules en gélatine, solutions, sirops, etc. De même, les compositions utilisables pour l'administration par voie parentérale, sont les formes pharmaceutiquement connues pour ce genre d'administration, par exemple des solutions, des suspensions ou émulsions aqueuses ou huileuses.
Pour l'administration par voie rectale, les compositions contenant les composés de l'invention se présentent géneralement sous forme de suppositoires.
Les formes pharmaceutiques telles que solutions injectables, suspensions injectables, comprimés, gouttes, suppositoires, etc. sont préparées selon les méthodes couramment utilisées par les pharmaciens. Les formes pharmaceutiques comprennent également les compositions qui permettent de délivrer le produit actif d'une manière progressive. On mélange les composés de l'invention avec un véhicule solide ou liquide, non toxique, pharmaceutiquement acceptable, et éventuellement avec un agent dispersant, un agent désintégrant, un agent stabilisant, etc. On peut y ajouter, le cas échéant, des agents édulcorants, des agents colorants, etc. Le pourcentage de produit actif dans les compositions pharmaceutiques peut varier dans des limites très larges, selon le patient et le mode d'administration, en particulier selon la fréquence d'administration.
Quant à la posologie journalière, elle peut varier dans une gamme étendue de doses unitaires, par exemple de 0,05 à 0,5 g de produit actif, selon le mode d'administration. Ainsi, par exemple, elle peut être de 0,1 à 0,5 g, de préférence 0,1 g, une à plusieurs fois par jour, lorsque le composé est administré sous forme de comprimé.
A titre d'exemple non limitatif d'une composition contenant un composé de formule I pouvant être administré par voie orale, on cite ci-après une formulation pour comprimés :

| | |
|---|---|
| composé 1 | 50 mg |
| méthylcellulose (Methocel K4M) | 200 mg |
| lactose sec | 154 mg |
| aérosil | 5 mg |
| acide citrique anhydre | 60 mg |
| talc | 11 mg |
| stéarate de magnésium | 20 mg |

## Revendications (Revendications pour l'(les) Etat(s) contractant(s) suivant(s): AT, BE, CH, DE, DK, ES, FR, GB, IT, LI, LU, MC, NL, PT, SE)

1. Cyclopropylamino-1,3,5-triazines substituées, leurs isomères optiques et leurs mélanges racémiques, répondant à la formule générale 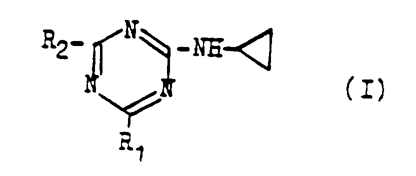 dans laquelle
R₁ représente un radical alkyle ou un radical cycloalkyle substitué ou non par au moins un radical alkyle, les radicaux alkyle ayant de 1 à 3 atomes de carbone et le radical cycloalkyle ayant de 3 à 5 atomes de carbone, et
R₂ représente le radical bis(2-hydroxyéthyl)amino, 3-hydroxy-1-azétidinyle, 3-méthoxy-1-azézidinyle, 3-oxo-1-azétidinyle, morpholino, 4-hydroxypipéridino, thiomorpholino, thiomorpholino S-oxyde, thiomorpholino S,S-dioxyde, 3-thiazolidinyle, 3-thiazolidinyle S-oxyde, 3-thiazolidinyle S,S-dioxyde ou 8-oxa-3-azabicyclo[3,2,1]oct-3-yle,
ainsi que leurs sels d'addition d'acides non toxiques pharmaceutiquement acceptables.

2. Cyclopropylamino-1,3,5-triazines répondant à la formule générale I donnée dans la revendication 1, dans laquelle R₂ représente le radical morpholino, thiomorpholino ou thiomorpholino S,S-dioxyde et leurs sels d'addition d'acides non toxiques pharmaceutiquement acceptables.

3. 2-cyclopropylamino-4-morpholino-6-n-propyl-1,3,5-triazine et ses sels d'addition d'acides non toxiques pharmaceutiquement acceptables.

4. 2-cyclopropyl-4-cyclopropylamino-6-morpholino-1,3,5-triazine et ses sels d'addition d'acides non toxiques pharmaceutiquement acceptables.

5. 2-cyclobutyl-4-cyclopropylamino-6-morpholino-1,3,5-triazine et ses sels d'addition d'acides non toxiques pharmaceutiquement acceptables.

6. 2-cyclopropyl-4-cyclopropylamino-6-thiomorpholino-1,3,5-triazine et ses sels d'addition d'acides non toxiques pharmaceutiquement acceptables.

7. 2-cyclopropyl-4-cyclopropylamino-6-thiomorpholino-1,3,5-triazine S,S-dioxyde et ses sels d'addition d'acides non toxiques pharmaceutiquement acceptables.

8. Procédé de préparation de cyclopropylamino-1,3,5-triazines substituées et de leurs sels tels que définis dans la revendication 1, caractérisé en ce que
a) on fait réagir une chloro-cyclopropylamino-1,3,5-triazine de formule 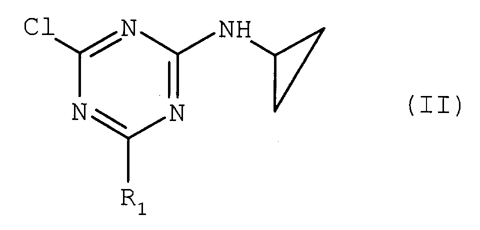 dans laquelle R₁ a la signification donnée à la revendication 1, avec une amine de formule R₂H (III) dans laquelle R₂ a la signification donnée à la revendication 1, ou on fait réagir une chloro-1,3,5-triazine de formule 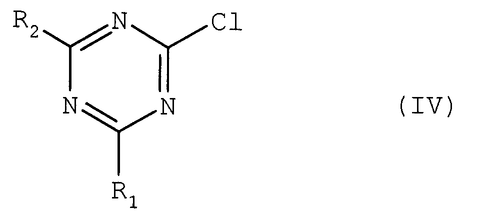 dans laquelle R₁ et R₂ ont la signification donnée à la revendication 1, avec la cyclopropylamine de formule 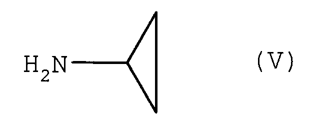 ou
b) on fait réagir un N-cyclopropylbiguanide de formule 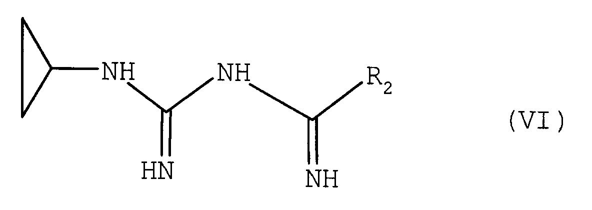 dans laquelle R₂ a la signification donnée à la revendication 1, avec un ester d'alkyle de formule R₁-COOAlk (VII) dans laquelle R₁ a la signification donnée à la revendication 1 et Alk représente un radical alkyle ayant de 1 à 4 atomes de carbone, et
c) en ce que, éventuellement, on convertit les cyclopropylamino-1,3,5-triazines de formule I ainsi obtenues en leurs sels d'addition d'acides non toxiques pharmaceutiquement acceptables.

9. Procédé de préparation de cyclopropylamino-1,3,5-triazines substituées et de leurs sels tels que définis dans la revendication 1 avec la caractéristique supplémentaire que R₂ représente le radical thiomorpholino S-oxyde, thiomorpholino S,S-dioxyde, 3-thiazolidinyle S-oxyde ou 3-thiazolidinyle S,S-dioxyde, caractérisé en ce qu'on oxyde une cyclopropylamino-1,3,5-triazine de formule 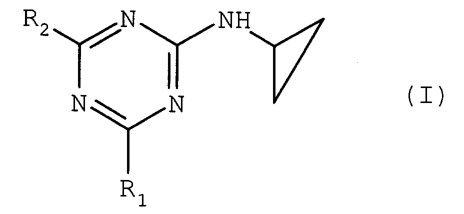 dans laquelle R₁ a la signification donnée à la revendication 1 et R₂ représente le radical thiomorpholino ou 3-thiazolidinyle, et en ce que, éventuellement, on convertit les cyclopropylamino-1,3,5-triazines de formule I ainsi obtenues en leurs sels d'addition d'acides non toxiques pharmaceutiquement acceptables.

10. Cyclopropylamino-1,3,5-triazines selon l'une quelconque des revendications 1 à 7 ou un de leurs sels d'addition d'acides non toxiques pharmaceutiquement acceptables pour utilisation dans le traitement des troubles associés à la maladie d'Alzheimer, aux démences séniles de type Alzheimer et à toute pathologie cognitive évolutive, dans le traitement de la dépression, de l'anxiété et des troubles de l'humeur, et dans le traitement des phénomènes inflammatoires et de l'asthme.

11. Compositions thérapeutiques renfermant une quantité thérapeutiquement efficace d'une cyclopropylamino-1,3,5-triazine selon l'une quelconque des revendications 1 à 7 ou d'un de ses sels d'addition d'acides non toxiques pharmaceutiquement acceptables, en association avec des excipients pharmaceutiques solides ou liquides.

## Revendications (Revendications pour l'(les) Etat(s) contractant(s) suivant(s): GR)

1. Procédé de préparation de cyclopropylamino-1,3,5-triazines substituées, leurs isomères optiques et leurs mélanges racémiques, répondant à la formule générale 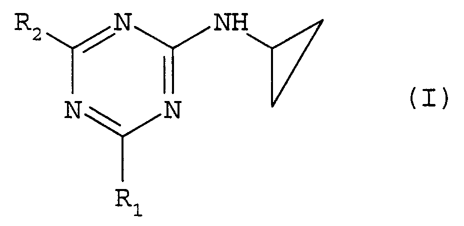 dans laquelle
R₁ représente un radical alkyle ou un radical cycloalkyle substitué ou non par au moins un radical alkyle, les radicaux alkyle ayant de 1 à 3 atomes de carbone et le radical cycloalkyle ayant de 3 à 5 atomes de carbone, et
R₂ représente le radical bis(2-hydroxyéthyl)amino, 3-hydroxy-1-azétidinyle, 3-méthoxy-1-azétidinyle, 3-oxo-1-azétidinyle, morpholino, 4-hydroxypipéridino, thiomorpholino, thiomorpholino S,S-oxyde, thiomorpholino S,S-dioxyde, 3-thiazolidinyle, 3-thiazolidinyle S-oxyde, 3-thiazolidinyle S,S-dioxyde ou 8-oxa-3-azabicyclo[3,2,1]oct-3-yle,
ainsi que leurs sels d'addition d'acides non toxiques pharmaceutiqument acceptables, caractérisés en ce que:
a) on fait réagir une chloro-cyclopropylamino-1,3,5-triazine de formule 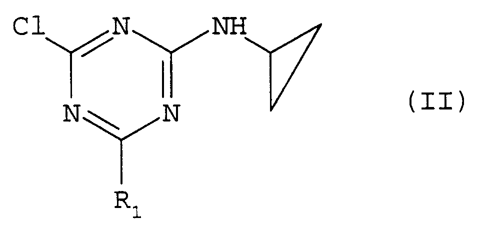 dans laquelle R₁ a la signification donnée ci-dessus avec une amine de formule R₂H (III) dans laquelle R₂ a la signification ci-dessus, ou on fait réagir une chloro-1,3,5-triazine de formule 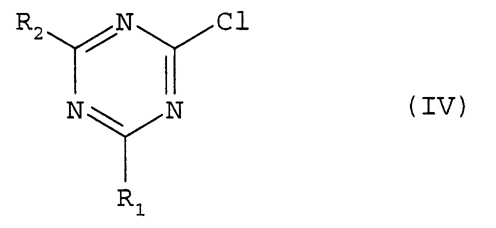 dans laquelle R₁ et R₂ ont la signification donnée ci-dessus, avec la cyclopropylamine de formule 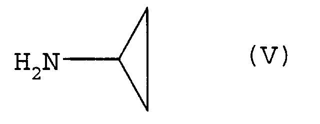 ou
b) on fait réagir un N-cyclopropylbiguanide de formule 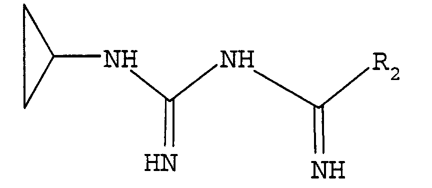 dans laquelle R₂ a la signification donnée ci-dessus, avec un ester d'alkyle de formule R₁-COOAlk (VII) dans laquelle R₁ a la signification donnée ci-dessus et Alk représente un radical alkyle ayant de 1 à 4 atomes de carbone, et
c) en ce que, éventuellement, on convertit les cyclopropylamino-1,3,5-triazines de formule I ainsi obtenues en leurs sels d'addition d' acides non toxiques pharmaceutiquement acceptables.

2. Procédé de préparation de cyclopropylamino-1,3,5-triazines substituées, leurs isomères optiques et leurs mélanges racémiques, répondant à la formule générale 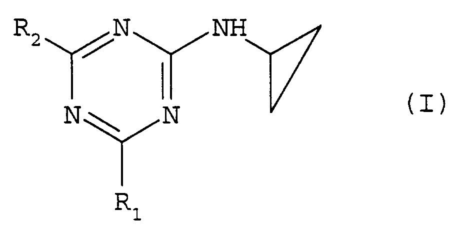 dans laquelle
R₁ représente un radical alkyle ou un radical cycloalkyle substitué ou non par au moins un radical alkyle, les radicaux alkyle ayant de 1 à 3 atomes de carbone et le radical cycloalkyle ayant de 3 à 5 atomes de carbone, et
R₂ représente le radical thiomorpholino S-oxyde, thiomorpholino S,S-dioxyde, 3-thiazolidinyle S-oxyde ou 3-thiazolidinyle S,S-dioxyde,
ainsi que leurs sels d'addition d'acides non toxiques pharmaceutiqument acceptables, caractérisé en ce qu'on oxyde une cyclopropylamino-1,3,5-triazine de formule 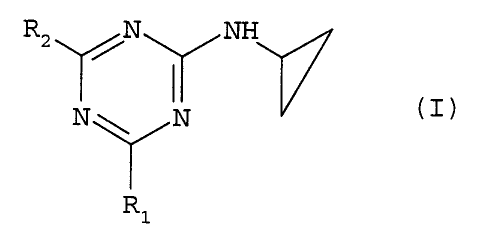 dans laquelle R₁ a la signification donnée ci-dessus et R₂ représente le radical thiomorpholino ou 3-thiazolidinyle, et en ce que, éventuellement, on convertit les cyclopropylamino-1,3,5-triazines de formule I ainsi obtenues en leurs sels d'addition d'acides non toxiques pharmaceutiquement acceptables.

## Claims (Claims for the following Contracting State(s): AT, BE, CH, DE, DK, ES, FR, GB, IT, LI, LU, MC, NL, PT, SE)

1. Substituted cyclopropylamino-1,3,5-triazines, their optically active isomers and racemic mixtures of the formula 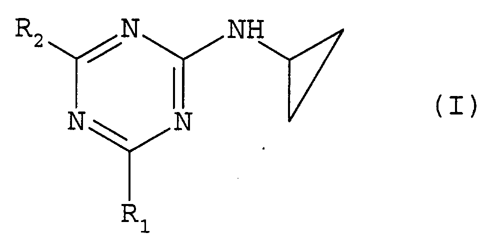 wherein
R₁ represents an alkyl radical, a substituted or unsubstituted cycloalkyl radical by at least one alkyl radical, the alkyl radicals having 1 to 3 carbon atoms and the cycloalkyl radicals having 3 to 5 carbon atoms, and
R₂ represents a bis(2-hydroxyethyl)amino, 3-hydroxy-1-azetidinyl, 3-methoxy-1-azetidinyl, 3-oxo-1-azetidinyl, morpholino, 4-hydroxypiperidino, thiomorpholino, thiomorpholino S-oxide, thiomorpholino S,S-dioxide, 3-thiazolidinyl, 3-thiazolidinyl S-oxide, 3-thiazolidinyl S,S-dioxide or 8-oxa-3-azabicyclo[3,2,1]oct-3-yl radical,
and their non-toxic pharmaceutically acceptable acid addition salts.

2. Cyclopropylamino-1,3,5-triazines having the general formula I given in claim 1, wherein R₂ represents a morpholino, thiomorpholino or thiomorpholino S,S-dioxide radical and their non-toxic pharmaceutically acceptable acid addition salts.

3. 2-cyclopropylamino-4-morpholino-6-n-propyl-1,3,5-triazine and its non-toxic pharmaceutically acceptable acid addition salts.

4. 2-cyclopropyl-4-cyclopropylamino-6-morpholino-1,3,5-triazine and its non-toxic pharmaceutically acceptable acid addition salts.

5. 2-cyclobutyl-4-cyclopropylamino-6-morpholino-1,3,5-triazine and its non-toxic pharmaceutically acceptable acid addition salts.

6. 2-cyclopropyl-4-cyclopropylamino-6-thiomorpholino-1,3,5-triazine and its non-toxic pharmaceutically acceptable acid addition salts.

7. 2-cyclopropyl-4-cyclopropylamino-6-thiomorpholino-1,3,5-triazine S,S-dioxide and its non-toxic pharmaceutically acceptable acid addition salts.

8. Process for the preparation of substituted cyclopropylamino-1,3,5-triazines and their salts as defined in claim 1, characterised in that
a) a chloro-cyclopropylamino-1,3,5-triazine of the formula 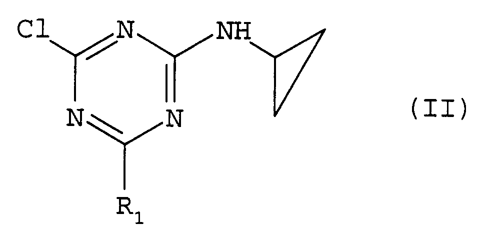 wherein R₁ has the meaning given in claim 1, is reacted with an amine of the formula R₂H (III), in which R₂ has the meaning given in claim 1; or a chloro-1,3,5-triazine of the formula 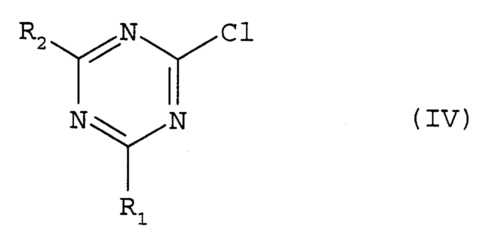 wherein R₁ and R₂ have the meanings given in claim 1, is reacted with the cyclopropylamine of the formula 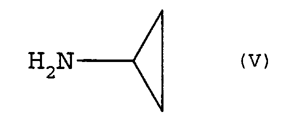
b) a N-cyclopropylbiguanide of the formula 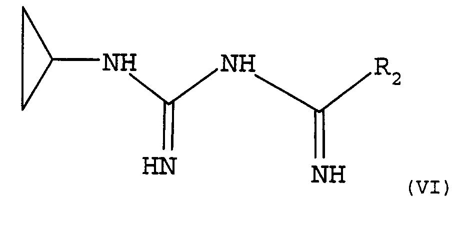 wherein R₂ has the meaning given in claim 1, is reacted with an alkyl ester of the formula R₁-COO Alk (VII), wherein R₁ has the meaning given in claim 1 and Alk represents an alkyl radical having 1 to 4 carbon atoms; and
c) in that, optionally, the cyclopropylamino-1,3,5-triazines of formula I so obtained are transformed into their non-toxic pharmaceutically acceptable acid addition salts.

9. Process for the preparation of a substituted cyclopropylamino-1,3,5-triazine and their salts as defined in claim 1, with the further characteristic that R₂ represents a thiomorpholino S-oxide, thiomorpholino S,S-dioxide, 3-thiazolidinyl S-oxide or 3-thiazolidinyl S,S-dioxide radical, characterised in that a cyclopropylamino-1,3,5-triazine of the formula 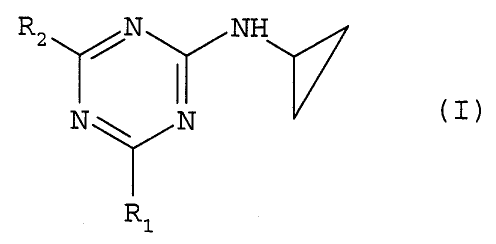 wherein R₁ has the meaning given in claim 1 and R₂ represents a thiomorpholino or 3-thiazolidinyl radical is oxidised, and in that, optionally, the cyclopropylamino-1,3,5-triazines of formula I so obtained are transformed into their non-toxic pharmaceutically acceptable acid addition salts.

10. Cyclopropylamino-1,3,5-triazines according to any one of the claims 1 to 7, or one of their non-toxic pharmaceutically acceptable acid addition salts for use in the treatment of disorders associated with Alzheimer's disease, with senile dementia of Alzheimer's type and with any evolutive cognitive pathology, for the treatment of depression, anxiety and mood, and for the treatment of inflammatory phenomena and asthma.

11. Therapeutic compositions comprising a therapeutically effective amount of a cyclopropylamino-1,3,5-triazine according to any one of the claims 1 to 7, of one of its non-toxic pharmaceutically acceptable acid addition salts, associated with solid or liquid pharmaceutical excipients.

## Claims (Claims for the following Contracting State(s): GR)

1. Process for the preparation of substituted cyclopropylamino-1,3,5-triazines, their optically active isomers and racemic mixtures of the general formula 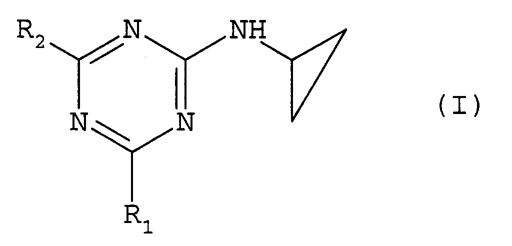 wherein
R₁ represents an alkyl radical, a substituted or unsubstituted cycloalkyl radical by at least one alkyl radical, the alkyl radicals having 1 to 3 carbon atoms and the cycloalkyl radicals having 3 to 5 carbon atoms, and
R₂ represents a bis(2-hydroxyethyl)amino, 3-hydroxy-1-azetidinyl, 3-methoxy-1-azetidinyl, 3-oxo-1-azetidinyl, morpholino, 4-hydroxypiperidino, thiomorpholino, thiomorpholino S-oxide, thiomorpholino S,S-dioxide, 3-thiazolidinyl, 3-thiazolidinyl S-oxide, 3-thiazolidinyl S,S-dioxide or 8-oxa-3-azabicyclo[3,2,1]oct-3-yl radical,
and their non-toxic pharmaceutically acceptable acid addition salts, characterised in that :
a) a chloro-cyclopropylamino-1,3,5-triazine of the formula 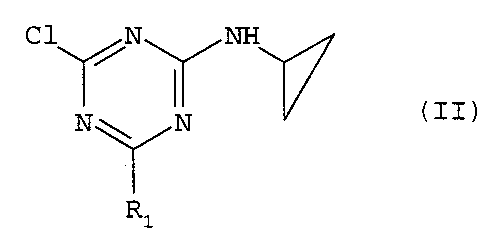 wherein R₁ has the meaning given in claim 1, is reacted with an amine of the formula R₂H (III), in which R₂ has the meaning given in claim 1; or a chloro-1,3,5-triazine of the formula 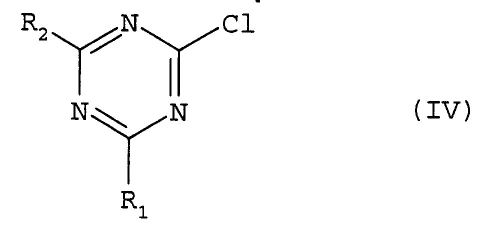 wherein R₁ and R₂ have the meanings given in claim 1, is reacted with the cyclopropylamine of the formula 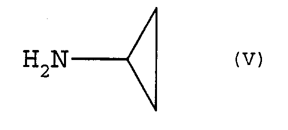 or
b) a N-cyclopropylbiguanide of the formula 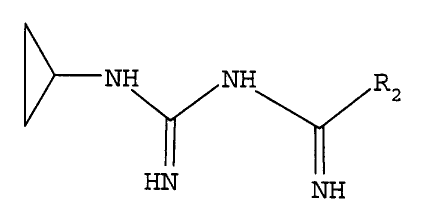 wherein R₂ has the meaning given in claim 1, is reacted with an alkyl ester of the formula R₁-COO Alk (VII), wherein R₁ has the meaning given in claim 1 and Alk represents an alkyl radical having 1 to 4 carbon atoms; and
c) in that, optionally, the cyclopropylamino-1,3,5-triazines of formula I so obtained are transformed into their non-toxic pharmaceutically acceptable acid addition salts.

2. Process for the preparation of substituted cyclopropylamino-1,3,5-triazines, their optically active isomers and racemic mixtures of the general formula 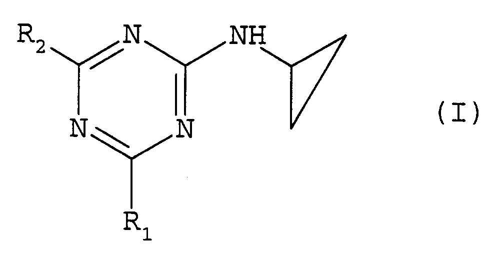 wherein
R₁ represents an alkyl radical, a substituted or unsubstituted cycloalkyl radical by at least one alkyl radical, the alkyl radicals having 1 to 3 carbon atoms and the cycloalkyl radicals having 3 to 5 carbon atoms, and
R₂ represents a thiomorpholino S-oxide, thiomorpholino S,S-dioxide, 3-thiazolidinyl S-oxide or 3-thiazolidinyl S,S-dioxide radical,
and their non-toxic pharmaceutically acceptable acid addition salts, characterised in that a cyclopropylamino-1,3,5-triazine of the formula 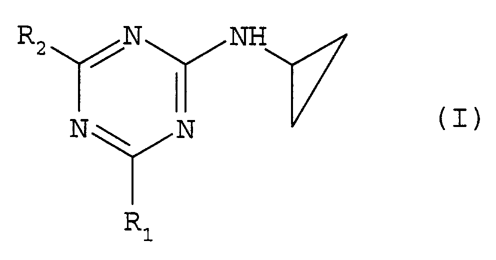 wherein R₁ has the meaning given hereabove and R₂ represents a thiomorpholino or 3-thiazolidinyl radical is oxidised, and in that, optionally, the cyclopropylamino-1,3,5-triazines of formula I so obtained are transformed into their non-toxic pharmaceutically acceptable acid addition salts.

## Patentansprüche (Patentansprüche für folgende(n) Vertragsstaat(en): AT, BE, CH, DE, DK, ES, FR, GB, IT, LI, LU, MC, NL, PT, SE)

1. Substituierte Cyclopropylamino-1,3,5-triazine, ihre optischen Isomere und ihre racemischen Gemische, die der allgemeinen Formel 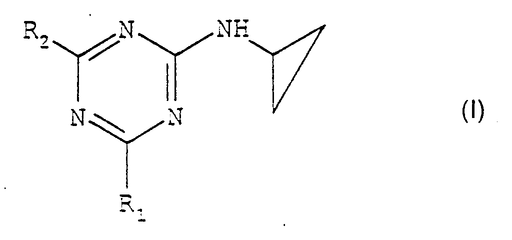 entsprechen, in der
R₁ einen Alkylrest oder einen mit mindestens einem Alkylrest substituierten oder nicht substituierten Cycloalkylrest darstellt, wobei die Alkylreste 1 bis 3 Kohlenstoffatome aufweisen und der Cycloalkylrest 3 bis 5 Kohlenstoffatome aufweist, und
R₂ den Bis(2-hydroxyethyl)amino-, 3-Hydroxy-1-azetidinyl-, 3-Methoxy-1-azetidinyl-, 3-Oxo-1-azetidinyl-, Morpholino-, 4-Hydroxypiperidino-, Thiomorpholino-, Thiomorpholino-S-oxid-, Thiomorpholino-S,S-dioxid-, 3-Thiazolidinyl-, 3-Thiazolidinyl-S-oxid-, 3-Thiazolidinyl-S,S-dioxid- oder 8-Oxa-3-azabicyclo[3,2,1]oct-3-yl-Rest darstellt,
sowie ihre Additions-Salze von nicht toxischen pharmazeutisch annehmbaren Säuren.

2. Cyclopropylamino-1,3,5-triazine, die der in Anspruch 1 angegebenen allgemeinen Formel I entsprechen, in der R₂ den Morpholino-, Thiomorpholino- oder Thiomorpholino-S,S-dioxid-Rest darstellt, und ihre Additions-Salze von nicht toxischen pharmazeutisch annehmbaren Säuren.

3. 2-Cyclopropylamino-4-morpholino-6-n-propyl-1,3,5-triazin und seine Additions-Salze von nicht toxischen pharmazeutisch annehmbaren Säuren.

4. 2-Cyclopropyl-4-cyclopropylamino-6-morpholino-1,3,5-triazin und seine Additions-Salze von nicht toxischen pharmazeutisch annehmbaren Säuren.

5. 2-Cyclobutyl-4-cyclopropylamino-6-morpholino-1,3,5-triazin und seine Additions-Salze von nicht toxischen pharmazeutisch annehmbaren Säuren.

6. 2-Cyclopropyl-4-cyclopropylamino-6-thiomorpholino-1,3,5-thiazin und seine Additions-Salze von nicht toxischen pharmazeutisch annehmbaren Säuren.

7. 2-Cyclopropyl-4-cyclopropylamino-6-thiomorpholino-1,3,5-triazin-S,S-dioxid und seine Additions-Salze von nicht toxischen pharmazeutisch annehmbaren Säuren.

8. Verfahren zur Herstellung substituierter Cyclopropylamino-1,3,5-triazine und ihrer Salze, wie in Anspruch 1 definiert, dadurch gekennzeichnet, daß
a) man ein Chlor-cyclopropylamino-1,3,5-triazin der Formel 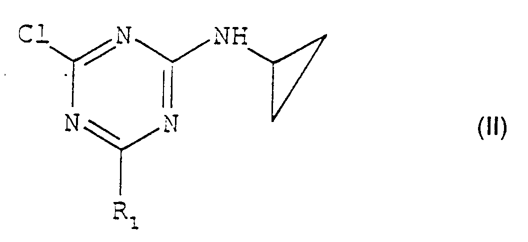 in der R₁ die in Anspruch 1 angegebene Bedeutung hat, mit einem Amin der Formel R₂H (III), in der R₂ die in Anspruch 1 angegebene Bedeutung hat, umsetzt, oder man ein Chlor-1,3,5-triazin der Formel 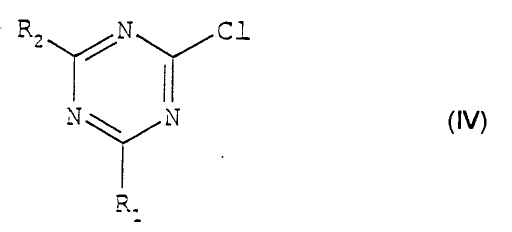 in der R₁ und R₂ die in Anspruch 1 angegebene Bedeutung haben, mit dem Cyclopropylamin der Formel 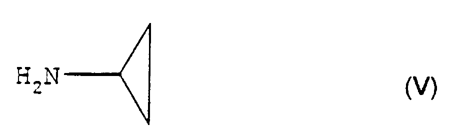 umsetzt,
b) man ein N-Cyclopropylbiguanid der Formel 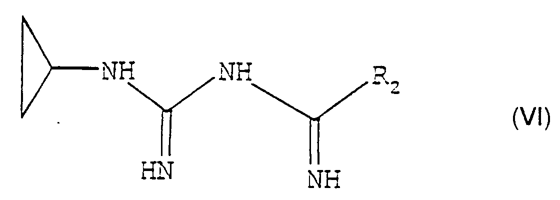 in der R₂ die in Anspruch 1 angegebene Bedeutung hat, mit einem Alkylester der Formel R₁-COOAlk (VII), in der R₁ die in Anspruch 1 angegebene Bedeutung hat und Alk einen Alkylrest mit 1 bis 4 Kohlenstoffatomen darstellt, umsetzt und
c) dadurch daß man gegebenenfalls die so erhaltenen Cyclopropylamino-1,3,5-triazine der Formel I in ihre Additions-Salze von nicht toxischen pharmazeutisch annehmbaren Säuren umwandelt.

9. Verfahren zur Herstellung substituierter Cyclopropylamino-1,3,5-triazine und ihrer Salze, wie in Anspruch 1 definiert, mit dem zusätzlichen Merkmal, daß R₂ den Thiomorpholino-S-oxid-, Thiomorpholino-S,S-dioxid-, 3-Thiazolidinyl-S-oxid oder 3-Thiazolidinyl-S,S-dioxid-Rest darstellt, dadurch gekennzeichnet, daß man ein Cyclopropylamino-1,3,5-triazin der Formel 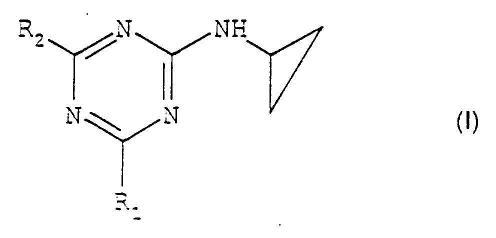 in der R₁ die in Anspruch 1 angegebene Bedeutung hat und R₂ den Thiomorpholino- oder 3-Thiazolidinylrest darstellt, oxidiert und dadurch, daß man gegebenenfalls die so erhaltenen Cyclopropylamino-1,3,5-triazine der Formel I in ihre Additions-Salze von nicht toxischen pharmazeutisch annehmbaren Säuren umwandelt.

10. Cyclopropylamino-1,3,5-triazine gemäß einem der Ansprüche 1 bis 7 oder eines ihrer Additions-Salze von nicht toxischen pharmazeutisch annehmbaren Säuren zur Verwendung bei der Behandlung von Störungen, die mit der Alzheimerschen Krankheit, mit senilen Demenzen vom Alzheimer-Typ und mit jeder fortschreitenden kognitiven Pathologie verbunden sind, bei der Behandlung von Depression, Angstzuständen und von Stimmungsstörungen und bei der Behandlung von entzündlichen Phänomenen und Asthma.

11. Therapeutische Zusammensetzungen, die eine therapeutisch wirksame Menge eines Cyclopropylamino-1,3,5-triazins gemäß einem der Ansprüche 1 bis 7 oder eines seiner Additions-Salze von nicht toxischen pharmazeutisch annehmbaren Säuren in Verbindung mit festen oder flüssigen pharmazeutischen Exzipienten enthalten.

## Patentansprüche (Patentansprüche für folgende(n) Vertragsstaat(en): GR)

1. Verfahren zur Herstellung substituierter Cyclopropylamino-1,3,5-triazine, ihrer optischen Isomere und ihrer racemischen Gemische, die der allgemeinen Formel 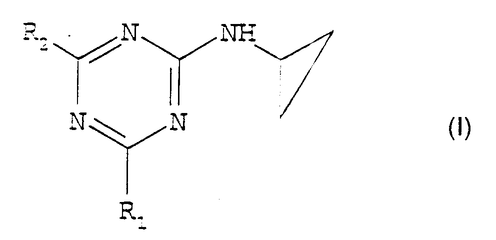 entsprechen, in der
R₁ einen Alkylrest oder einen mit mindestens einem Alkylrest substituierten oder nicht substituierten Cycloalkylrest darstellt, wobei die Alkylreste 1 bis 3 Kohlenstoffatome aufweisen und der Cycloalkylrest 3 bis 5 Kohlenstoffatome aufweist, und
R₂ den Bis(2-hydroxyethyl)amino, 3-Hydroxy-1-azetidinyl-, 3-Methoxy-1-azetidinyl-, 3-Oxo-1-azetidinyl-, Morpholino-, 4-Hydroxypiperidino-, Thiomorpholino-, Thiomorpholino-S-oxid-, Thiomorpholino-S,S-dioxid, 3-Thiazolidinyl-, 3-Thiazolidinyl-S-oxid-, 3-Thiazolidinyl-S,S-dioxid- oder 8-Oxa-3-azabicyclo[3,2,1]oct-3-yl-Rest darstellt,
sowie ihrer Additions-Salze von nicht toxischen pharmazeutisch annehmbaren Säuren, dadurch gekennzeichnet, daß:
a) man ein Chlor-cyclopropylamino-1,3,5-triazin der Formel 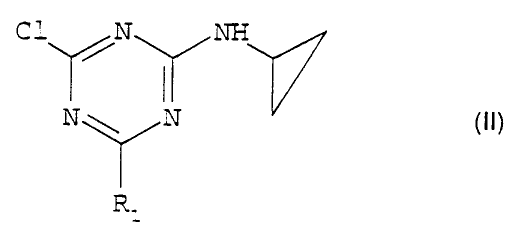 in der R₁ die oben angegebene Bedeutung hat, mit einem Amin der Formel R₂H (III), in der R₂ die oben angegebene Bedeutung hat, umsetzt, oder man ein Chlor-1,3,5-triazin der Formel 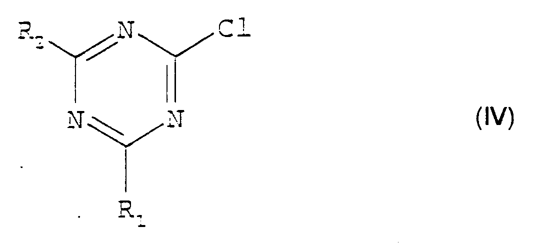 in der R₁ und R₂ die oben angegebene Bedeutung haben, mit dem Cyclopropylamin der Formel 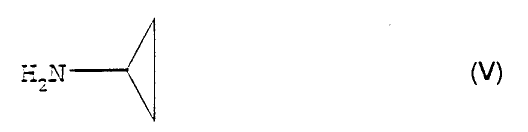 umsetzt oder
b) man ein N-Cyclopropylbiguanid der Formel 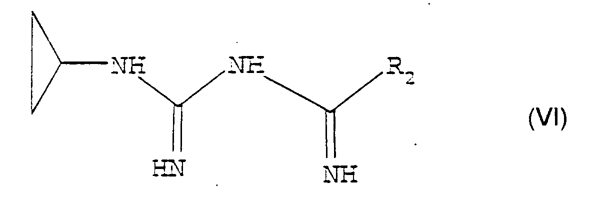 in der R₂ die in oben angegebene Bedeutung hat, mit einem Alkylester der Formel R₁-COOAlk (VII), in der R₁ die oben angegebene Bedeutung hat und Alk einen Alkylrest mit 1 bis 4 Kohlenstoffatomen darstellt, umsetzt und
c) dadurch, daß man gegebenenfalls die so erhaltenen Cyclopropylamino-1,3,5-triazine der Formel I in ihre Additions-Salze von nicht toxischen pharmazeutisch annehmbaren Säuren umwandelt.

2. Verfahren zur Herstellung substituierter Cyclopropylamino-1,3,5-triazine, ihrer optischen Isomere und ihrer racemischen Gemische, die der allgemeinen Formel 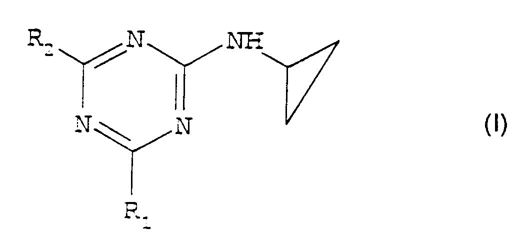 entsprechen, in der
R₁ einen Alkylrest oder einen mit mindestens einem Alkylrest substituierten oder nicht substituierten Cycloalkylrest darstellt, wobei die Alkylreste 1 bis 3 Kohlenstoffatome aufweisen und der Cycloalkylrest 3 bis 5 Kohlenstoffatome aufweist, und
R₂ den Thiomorpholino-S-oxid-, Thiomorpholino-S,S-dioxid-, 3-Thiazolidinyl-S-oxid- oder 3-Thiazolidinyl-S,S-dioxid-Rest darstellt,
sowie ihrer Additions-Salze von nicht toxischen pharmazeutisch annehmbaren Säuren, dadurch gekennzeichnet, daß man ein Cyclopropylamino-1,3,5-triazin der Formel 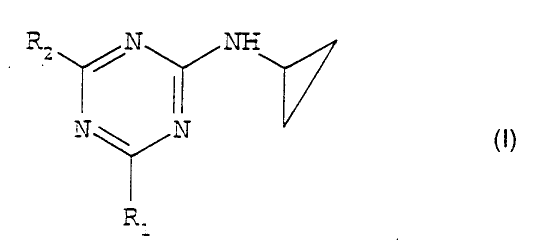 in der R₁ die oben angegebene Bedeutung hat und R₂ den Thiomorpholino- oder 3-Thiazolidinylrest darstellt, oxidiert und dadurch, daß man gegebenenfalls die so erhaltenen Cyclopropylamino-1,3,5-triazine der Formel I in ihre Additions-Salze von nicht toxischen pharmazeutisch annehmbaren Säuren umwandelt.
